# EUROPEAN PATENT SPECIFICATION

(11) **EP 3 946 283 B1**
(45) Date of publication and mention of the grant of the patent: **09.12.2026**
(21) Application number: 20784801.1
(22) Date of filing: 30.03.2020
(51) Int. Cl.: A61K 9/70, A61K 35/39, A61K 47/36, A61K 47/42, A61P 3/10

(54) **THERAPEUTIC HYDROGEL DEVICE**
VORRICHTUNG FÜR THERAPEUTISCHES HYDROGEL
DISPOSITIF D'HYDROGEL THÉRAPEUTIQUE

(30) Priority: 29.03.2019 SG 10201902844S
(43) Date of publication of application: 09.02.2022
(60) Divisional application: 26216252.2
(73) Proprietor: Nanyang Technological University, Singapore 639798 (SG)
(72) Inventor: DANG, Thuy Tram, Singapore 639798 (SG); CHEN, Yang, Singapore 639798 (SG); NGUYEN, Tri Dang, Singapore 639798 (SG); ZUO, Yicong, Singapore 639798 (SG)
(74) Representative: Mewburn Ellis LLP
(86) International application number: PCT/SG2020/050193
(87) International publication number: WO 2020/204829

(56) References cited:
- WO-A1-2018/055452
- WO-A1-2020/168327
- US-A1- 2013 137 155
- US-B2- 7 427 415
- US-B2- 9 422 524
- JONG MIN LEE ET AL: "Uniform-sized neurosphere-mediated motoneuron differentiation in microwell arrays", ELECTROPHORESIS, VERLAG CHEMIE, HOBOKEN, USA, vol. 38, no. 24, 6 September 2017 (2017-09-06), pages 3161 - 3167, XP071504458, ISSN: 0173-0835, DOI: 10.1002/ELPS.201700118
- IWASE M. ET AL.: "Formation of Cell Aggregates Using Microfabricated Hydrogel Chambers for Assembly into Larger Tissues", J. ROBOT. MECHATRON., vol. 25, no. 4, 20 August 2013 (2013-08-20), pages 682 - 689, XP055745436, [retrieved on 20200721]
- NAPOLITANO A. P. ET AL.: "Dynamics of the Self-Assembly of Complex Cellular Aggregates on Micromolded Nonadhesive Hydrogels", TISSUE ENGINEERING, vol. 13, no. 8, 14 August 2007 (2007-08-14), pages 2087 - 2094, XP002494838, [retrieved on 20200721], DOI: 10.1089/ten.2006.0190
- CHEN Y. ET AL.: "Microencapsulated islet-like microtissues with toroid geometry for enhanced cellular viability", ACTA BIOMATERIALIA, vol. 97, 9 August 2019 (2019-08-09), pages 260 - 271, XP085861888, [retrieved on 20200721], DOI: 10.1016/j.actbio.2019.08.018

## Description

### FIELD OF THE INVENTION

The present disclosure generally relates to a therapeutic hydrogel device. More particularly, the present disclosure describes various embodiments of a hydrogel macrodevice, such as a planar hybrid hydrogel macrodevice that can achieve spatially controlled distribution of microtissues and support establishment of intra-device vasculature for enhanced cell survival, and individually encapsulated microtissues, and methods of use.

### BACKGROUND OF THE INVENTION

Clinical islet transplantation is a potential strategy to recapitulate the physiological dynamics of glucose-responsive insulin secretion from native pancreatic β cells for the treatment of type 1 diabetes [Hering, B.J. et al., Diabetes Care 39 (7): 1230-1240 (2016); Shapiro, A.M. et al., Nat Rev Endocrinol 13 (5): 268-277 (2017)]. Encapsulating islet grafts in semi-permeable hydrogel membrane has been postulated as a promising approach to protect the donor cells from immunological attack by the host immune system [Desai, T. et al., Nat Rev Drug Discov 16 (5): 338-350 (2017); Lim, F. and Sun, A.M. Science 210 (4472): 908-910 (1980)]. This encapsulation approach allows for effective nutrient and waste exchange as well as the delivery of insulin to the host systemic circulation while enabling the expanded use of non-autologous cell sources to reduce the requirement for donor organs and eliminating the need for immunosuppressants [Desai, T. et al., Nat Rev Drug Discov 16 (5): 338-350 (2017)]. However, poor viability and limited long-term function of encapsulated islets hindered the clinical translation of this treatment strategy [Tuch, B.E. et al., Diabetes care 32 (10): 1887-1889 (2009); de Groot, M. et al., J Surg Res 121 (1): 141-150 (2004)]. Native islets, which account for 1-2% of the human adult pancreas volume [Saisho, Y. Rev Diabet Stud 13 (2-3): 132 (2016)] but receive ~10% of its blood flow [Jansson, L. and Hellerstrom, C. Am J Physiol 251 (6 Pt 1): E644-647 (1986)], are spheroidal cell clusters with mean diameters ranging from 50-500 µm [Kim, A. et al., Islets 1 (2): 129-136 (2009); Kilimnik, G. et al., Islets 4 (2): 167-172 (2012)]. In the pancreatic niche, which is actively supported by a rich capillary network, native islet size and geometry are sufficient to support islet survival and function [lonescu-Tirgoviste, C. et al., Sci Rep 5: 14634 (2015)]. However, after transplantation into a recipient, encapsulated islets are deprived of their supporting blood vessels and rely mainly on passive diffusion for metabolic exchange [de Groot, M. et al., J Surg Res 121 (1): 141-150 (2004)]. Consequently, after transplantation, encapsulated pancreatic islets often suffer from poor diffusion-limited transport of nutrients and oxygen, resulting in hypoxia and associated tissue death at the islet core [de Groot, M. et al., J Surg Res 121 (1): 141-150 (2004)]. This significant decrease in islet viability leads to the eventual failure of glycemic control and loss of insulin independence in the long term [Köllmer, M. et al., Tissue Eng Part B Rev 22 (1): 34-46 (2015); Elliott, R.B. et al., Xenotransplantation 14 (2): 157-161 (2007)].

Diffusion chambers or cell-laden hydrogel sheets have been evaluated in various preclinical studies for islet transplantation with variable outcome [Desai, T. Shea, LD. Nat. Rev. Drug Discov. 16(5): 338 (2017); Boettler, T. et al., Cell Transplant. 25(3): 609-614 (2016); Bruni, A. et al., Diabetes, metabolic syndrome and obesity: targets and therapy. 7: 211 (2014); Lee, BR. et al., Biomaterials. 33(3): 837-845 (2012)]. However, the high cell packing density, which is required to achieve sufficient dosage for therapeutic correction, increases the possibility of cell or microtissue aggregation and their nonhomogeneous spatial distribution leading to limited mass transfer, subsequent hypoxia and associated tissue necrosis as well as eventual impaired function of the therapeutic cells [Colton, CK. Advanced drug delivery reviews. 67: 93-110 (2014); Avgoustiniatos, ES. Colton, CK. Annals of the New York Academy of Sciences. 831(1 Bioartificial Organs: Science, Medicine, and Technology): 145-166 (1997)].

Another factor implicated in the limited success of these existing devices is the loss of native vasculature supporting the transplanted cells after they were isolated from donor sources, resulting in poor oxygen and nutrient supply and eventual tissue necrosis [Song, S. Roy, S. Biotechnol Bioeng. 113(7): 1381-1402 (2016)]. Generally, after device implantation, a new vascular network will start to invade the device as part of the host wound healing response. However, this process of forming new blood vessels from an existing vascular network, also known as angiogenesis, is slow and often produces less blood vessels than the vascular network surrounding the islets in the native pancreatic niche [Rouwkema J, Khademhosseini A. Trends Biotechnol. 34(9): 733-745 (2016)]. This problem is exacerbated by the monolithic structure and macroscopic dimension of the devices preventing new host vasculature from reaching the encapsulated islets, particularly those at the center.

Multiple strategies have been attempted to overcome hypoxia-induced cell death [Barkai, U. et al., Cell Transplant 22 (8): 1463-1476 (2013); Ramachandran, K. et al., Tissue Eng Part A 19 (5-6): 604-612 (2012)]. For example, revascularization approaches involve the co-encapsulation of islets with angiogenic genes, growth factors and vascular-inductive cell types to facilitate the formation of new vasculature and subsequently improve transport of oxygen to the transplanted grafts [Barkai, U. et al., Cell Transplant 22 (8): 1463-1476 (2013)]. More recently, a 3D bio-printing technique, involving photopolymerizable hydrogels and projection stereolithography, to generate a vasculature system was developed [Grigoryan, B. et al., Science 364: 458 (2019)]. This design was employed to engineer a vascular network-embedded device for delivery of hepatocyte aggregates to a rodent model of chronic liver injury, and the engraftment was proved to survive and be functional after 14 days post-transplantation. Another alternative strategy is to infuse exogenous oxygen gas into islet-containing macroencapsulation device via subdermally implanted access ports [Ramachandran, K. et al., Tissue Eng Part A 19 (5-6): 604-612 (2012)]. These approaches are limited by the requirements for additional biochemical agents and operational accessories, which complicate the islet delivery system and render its clinical translation more difficult. In addition, several studies have proposed to re-aggregate single cells obtained by collagenase dispersion of isolated islets to form spheroidal cell clusters with smaller diameters of approximately 50-100 µm [O'Sullivan, E. et al., Diabetologia 53 (5): 937-945 (2010); Hilderink, J. et al., J Cell Mol Med 19 (8): 1836-1846 (2015); Bosco, D. et al., Exp Cell Res 184 (1): 72-80 (1989)]. Even though smaller aggregates exhibited enhanced cellular viability compared to that of large islets, they suffer from decreased glucose-responsiveness due to poorer cell-cell interaction [Mendelsohn, A.D. et al., Acta Biomater 8 (12): 4278-4284 (2012); Wilson J.T. and Chaikof, E.L. Adv Drug Del Rev 60 (2): 124-145 (2008)]. Furthermore, for the same number of transplanted cells, using small aggregates necessitates a higher number of microtissues and subsequently an increased number of hydrogel microcapsules for encapsulation, resulting in a larger transplant volume [Reynolds, T.D. et al., Drug Dev Ind Pharm 28 (4): 457-466 (2002)]. Nonetheless, these studies have focused solely on microtissues with spheroidal geometry while the potential role of other non-spheroidal geometry on microtissue viability remains largely unexplored.

Of all convex shapes, spheroidal geometry has been theoretically proven to have the lowest SA/Vol for a given volume [Komatsu, H. et al., PloS one 12 (8): e0183780 (2017)]. Furthermore, Mullen *et al* has proven that a spheroidal human islet has a theoretical maximum limit on its radius to maintain cell viability [Enmon Jr, R.M. et al., Biotechnol Bioeng 72 (6): 579-591 (2001); Dean, D.M. et al., FASEB J 21 (14): 4005-4012 (2007)], rendering spheroidal geometry less favorable for diffusion-driven metabolic exchange. In contrast, it has been postulated that a rod-like microtissue has no theoretical limit of its length as long as its radius is within the critical diffusion limit required to support cell survival [Rago, A.P. et al., Tissue Eng Part A 15 (2): 387-395 (2008)]. Another appealing geometry is the toroid due to its lumen-containing structure which decreases the distance for the oxygen and media to diffuse into the interior cells [Livoti C.M. and Morgan, J.R. Tissue Eng Part A 16 (6): 2051-2061 (2010); Merglen, A. et al., Endocrinology 145 (2): 667-678 (2004)]. There remains a need to develop a therapeutic encapsulated microtissue system in which there is at least an improvement and/or advantage over the prior art.

US2013/137155 describes molds for making cell aggregation devices including upper and lower surfaces and methods of aggregating cells including depositing cells in a seeding chamber, incubating the cells and removing aggregated cells.

### SUMMARY OF THE INVENTION

The invention is as defined in the appended claims.

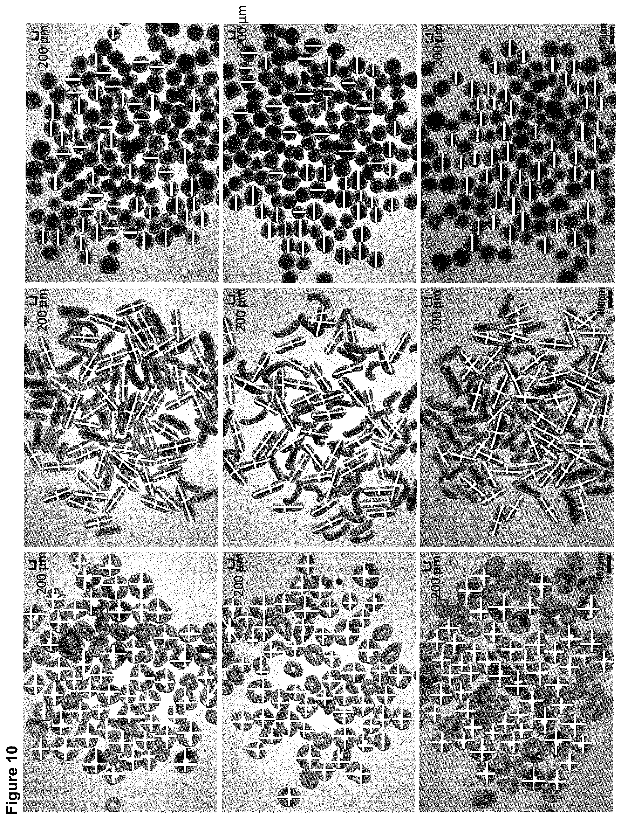

As disclosed herein, a dedicated platform was developed to encapsulate therapeutic microtissues with controlled spatial distribution while optionally concurrently supporting an organized intra-device network of vascular-inductive cells. This platform comprises two modules with complementary topography features that fit together in a lock-and-key configuration (Fig. 10). Inspired by the grid-like pattern on the surface of a waffle, the "lock" component consists of an interconnected vascular-supporting hydrogel network, which serves as dividing sidewalls separating evenly spaced microwells. The "key" component consists of therapeutic microtissues encapsulated in an immuno-isolatory hydrogel that is added to the microwells of the lock component and cross-linked, thereby essentially fixing or locking the components together as a unit. The waffle-inspired micropattern of the lock component was designed to direct the spatially homogenous distribution of therapeutic microtissues such that each microtissue can be ideally entrapped in one microwell to prevent undesirable aggregation. Furthermore, the interlocking design of the device minimizes the risk of the two components detaching and to ensure the proximity of optional vascular-inductive cells in the walls of the lock component and the encapsulated therapeutic microtissues in the key component.

According to a first aspect of the present disclosure, there is a planar biocompatible hydrogel-based macrodevice comprising an array of microwells and a therapeutic microtissue within each of a plurality of said microwells, wherein;
a) said macrodevice comprises a component comprising an interconnected network of cross-linked hydrogel, which serves as dividing sidewalls separating evenly spaced microwells, wherein each microwell in the array comprises at least one side wall and is configured to encapsulate a single therapeutic microtissue; and
b) said macrodevice comprises a component comprising therapeutic microtissues in an immuno-isolatory hydrogel that, when cross-linked, are entrapped in the microwells, and

wherein said immuno-isolatory hydrogel component of b) interlocks with the interconnected hydrogel network component of a); and wherein
the planar biocompatible hydrogel-based macrodevice further comprises an encapsulating coating of an immuno-isolatory hydrogel.

In some embodiments the at least one side wall of each of a plurality of said microwells further comprise vascular endothelial cells.

In some embodiments the therapeutic microtissues are toroid-shaped.

According to a second aspect of the present disclosure, there is a method of manufacturing a planar biocompatible hydrogel-based macrodevice, comprising the steps;
a) hydrogel prepolymer is dispensed onto a surface;
   i) a photomask with an array of holes or transparent features of specified geometry and dimensions is placed over the dispensed mixture, and
   ii) the mixture is exposed to UV light or solution containing cross-linker to crosslink an exposed portion of the hydrogel prepolymer, to form a micropatterned network of sidewalls, and
   iii) the crosslinked hydrogel pattern is rinsed to remove non-crosslinked hydrogel residue, leaving behind microwells; or
b) hydrogel prepolymer is dispensed onto a surface using 3D printing and cross-linked to form a micropatterned network of sidewalls defining microwells;
c) adding a mixture of microtissues and cross-linkable immuno-isolating hydrogel onto the crosslinked hydrogel micropattern of a) or b), and crosslinking the immunoisolating hydrogel; or
d) adding a suspension of cells to the microwells and culturing the cells under suitable conditions until the cells aggregate into desired microtissue shape depending on the chosen cross-linked hydrogel micropattern, and
   (i) adding cross-linkable immuno-isolating hydrogel onto the cross-linked hydrogel micropattern, and
   (ii) crosslinking the immuno-isolating hydrogel,
wherein the planar biocompatible hydrogel-based macrodevice comprises a single microtissue per microwell.

The method further comprises a step e)i) of encapsulating the planar biocompatible hydrogel-based macrodevice with a coating of a cross-linkable immuno-isolatory hydrogel and optionally e)ii) crosslinking the immuno-isolating hydrogel.

According to a third aspect of the present disclosure, there is a composition comprising cells for implantation, wherein the composition comprises a biocompatible hydrogel-based microcapsule having a single toroid-shaped microtissue encapsulated therein, wherein said microtissue secretes a therapeutically effective substance, such as a hormone or protein. In some embodiments the hydrogel is alginate

According to a fourth aspect of the present disclosure, there is a planar biocompatible hydrogel for use in a method of treatment comprising implanting into a subject in need of such treatment a planar biocompatible hydrogel-based macrodevice of any aspect of the disclosure or a composition of any aspect of the disclosure.

According to a fifth aspect of the present disclosure, there is a planar biocompatible hydrogel-based macrodevice of any aspect of the disclosure or a composition of any aspect of the disclosure for use as an implant for treating a subject

According to a sixth aspect of the present disclosure, there is a kit comprising a planar biocompatible hydrogel-based macrodevice of any aspect of the disclosure and a plurality of microtissues and/or alginate.

### BRIEF DESCRIPTION OF THE FIGURES

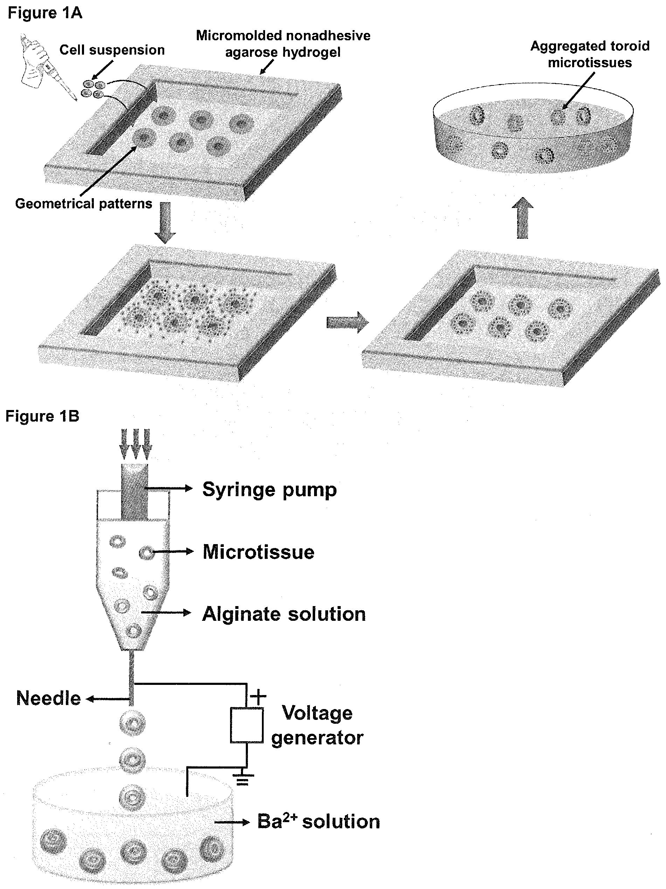

**Figures 1A-B** are schematic diagrams of the procedure to fabricate microtissues. **Fig. 1A** is a schematic diagram of the procedure to fabricate INS-1E microtissues using micromolded nonadhesive agarose hydrogels. Homogeneous suspension of dispersed cells was seeded into each micromolded nonadhesive agarose hydrogel by pipetting. After at least 55 hours of cellular assembly, microtissues were retrieved using a micropipette. **Fig. 1B** is a schematic diagram of the procedure to encapsulate islet-like microtissues using electrostatic droplet generator. A mixture of microtissues/alginate solutions was extruded through the 20G blunt needle into the bath of Ba²+ ions to form hydrogel microcapsules.

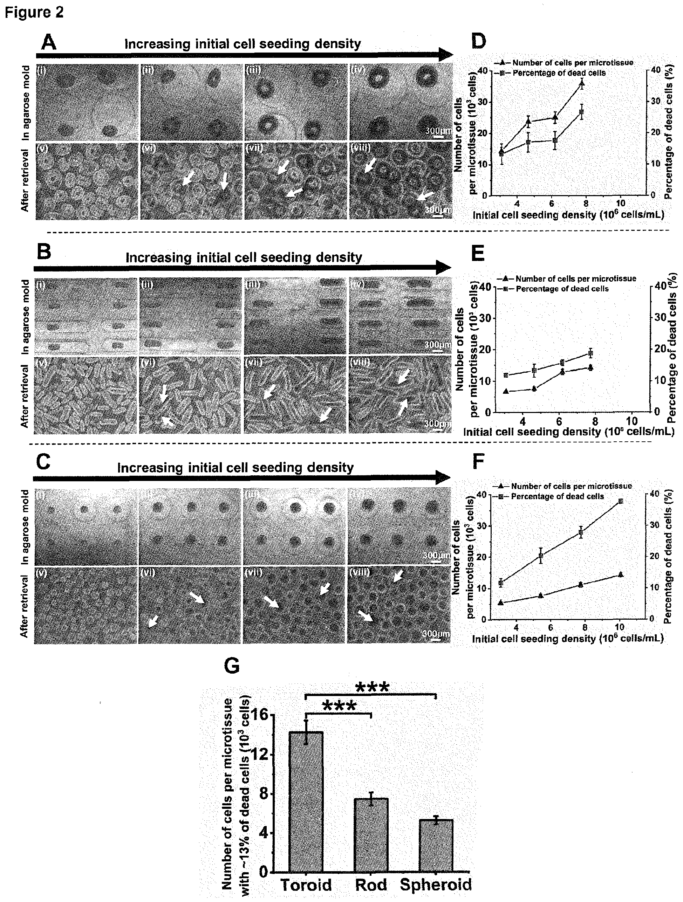

**Figure 2A-G** shows the effect of initial cell seeding density on cell number and viability of microtissues. **Fig. 2A****-C:** Optical images of microtissues with toroid, rod and spheroid geometries before **(i-iv)** and after **(v-viii)** retrieval from the micromolded agarose hydrogels. The arrows indicate dark regions of dead cells at the core of microtissues; **Fig. 2D****-F:** Quantitative assay using automated cell counter and trypan blue exclusion indicated that for each geometry, the number of cells per microtissue and the percentage of dead cells increased with initial cell seeding density. **Fig. 2G****:** Toroid microtissues with approximately 13% dead cells had a higher number of cells per microtissue than that of rod and spheroid microtissues with a similar percentage of dead cells. Data is shown as mean ± SEM (n=3 replicate samples). (***) denotes p < 0.005. The scale bars representing 300 µm apply to all images acquired at the same magnification.

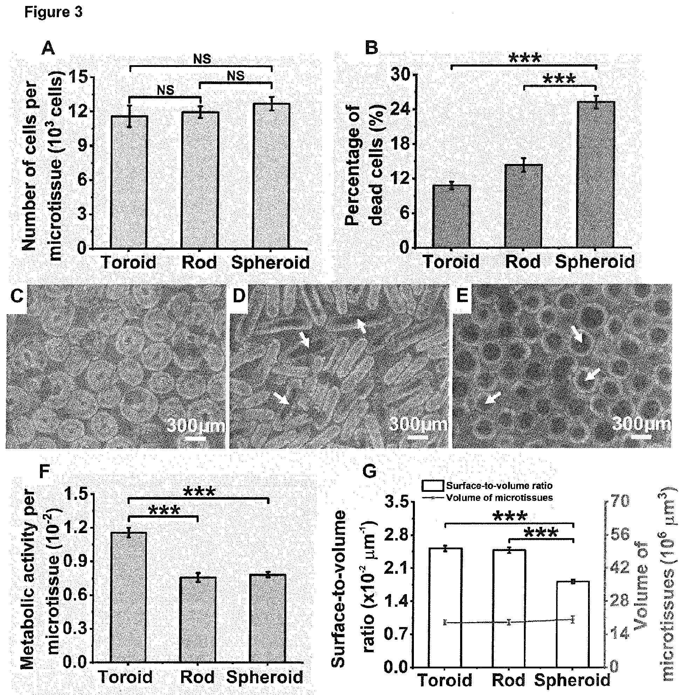

**Figure 3A-G** shows the effect of microtissue geometry on cellular viability. **Fig. 3A** : Quantitative assay with automated cell counter confirmed the average number of cells per microtissue was similar for all three geometries (n=6 replicate samples); **Fig. 3B** Toroid and rod microtissues had a lower percentage of dead cells compared to that of spheroid microtissues (n=6 replicate samples); **Fig. 3C-E** Optical images of microtissues with a standardized number of cells (~12000 cells) per microtissue. The arrows indicate dark regions of dead cells at the core of rod and spheroid microtissues; **Fig. 3F** Quantification of metabolic activity of intact microtissues with WST-1 assay (n=10 replicate samples) indicated that toroid microtissues were more metabolically active than rod and spheroid microtissues; **Fig. 3G** Volume and SA/Vol ratio of microtissues (n=3 batches of microtissues, 53 microtissues per batch). Data is shown as mean ± SEM (***) denotes p < 0.005. All scar bars represent 300 µm.

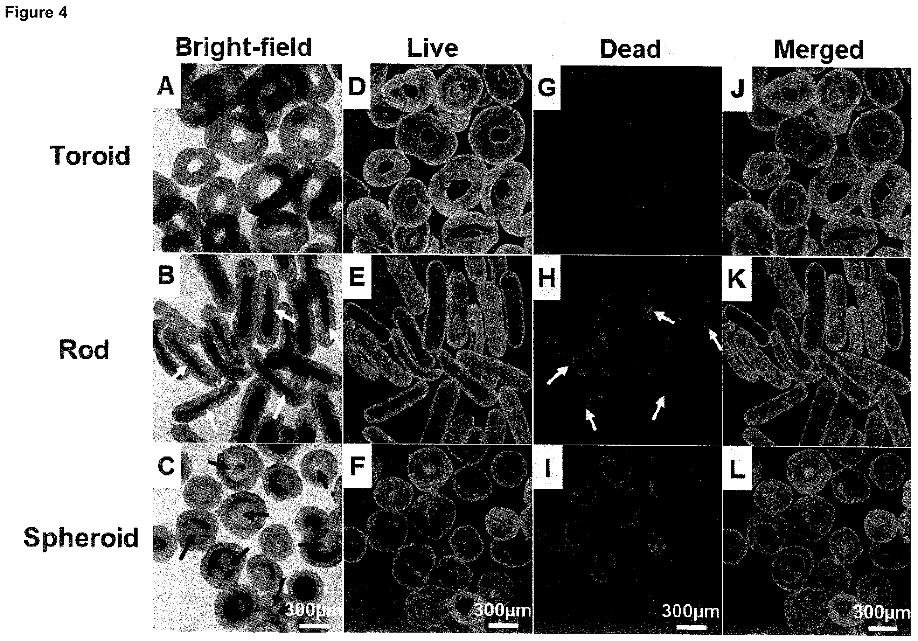

**Figure 4A-L** shows assessment of viability of microtissues by live/dead staining and confocal microscopy. **Fig. 4A****-C:** Bright-field images of microtissues. **Fig. 4D-F:** Fluorescence indicated live cells of microtissues; **Fig. 4G****-I:** Fluorescence indicates dead cells of microtissues; **Fig. 4J-L:** Merged images of live and dead cells of microtissues. White arrows in **Fig. 4B** and **H** indicate regions of dead cells in the interior of rod microtissues while black arrows in **Fig. 4C** indicate spheroid microtissues with dislodged dead cells after staining; All scale bars represent 300 µm.

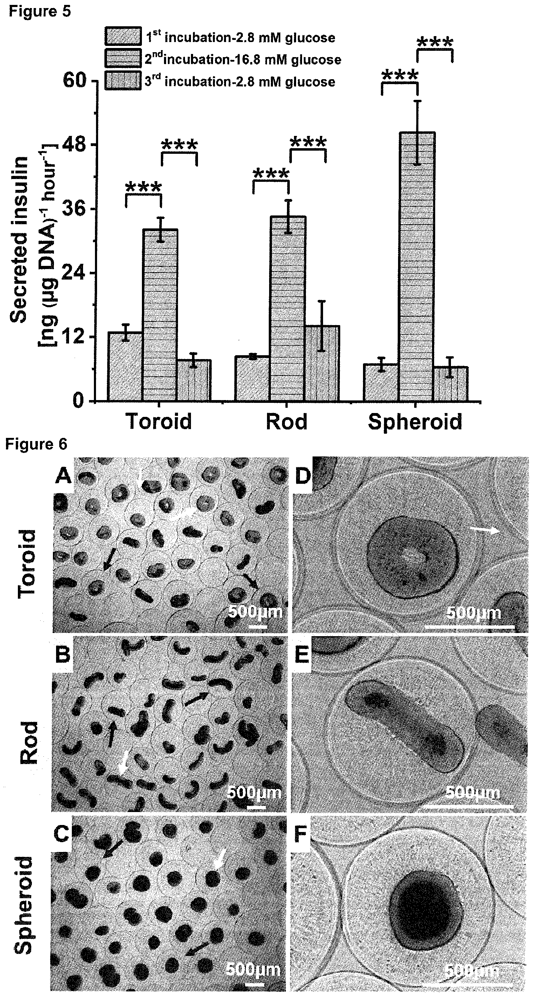

**Figure 5** shows insulin secretion from microtissues in response to static glucose stimulation. Insulin secretion from microtissues in response to static glucose stimulation. Microtissues were consecutively incubated in low glucose (2.8 mM) and high glucose (16.8 mM) followed by low glucose (2.8 mM) for 1 h at each concentration. For each geometry, a higher amount of insulin was secreted in response to the higher glucose level. Data is shown as mean ± SEM (n=6 replicate samples). (*) and (***) denote p-values less than 0.05 and 0.005 respectively.
**Figure 6A-F** shows optical images of microtissues encapsulated in alginate hydrogel capsules. Optical images of multiple **(****Fig. 6A-C)** and single **(****Fig. 6D-F)** toroid, rod and spheroid microtissues encapsulated in the alginate microcapsules. The black arrows indicate the surface of the uniform spherical alginate microcapsules which were optically transparent. The white arrows indicate the exterior surface of the microtissues. All scale bars represent 500 µm.

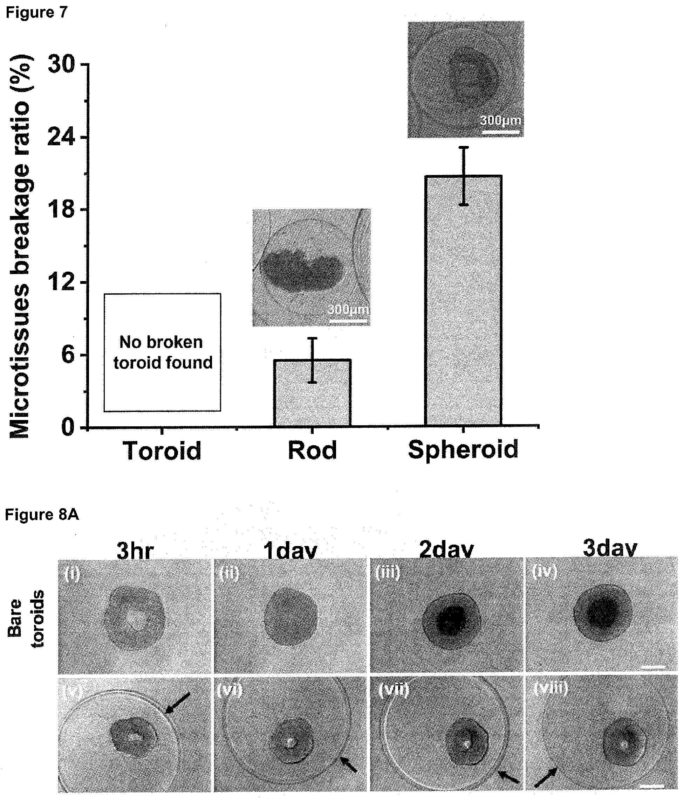

**Figure 7** shows quantification of breakage ratio during microencapsulation of microtissues of toroid, rod and spheroid geometries. Data is shown as mean ± SEM from two independent experiments. Approximately 115 to 155 microtissues were examined for each geometry in each independent experiment. (***) denotes p < 0.005.

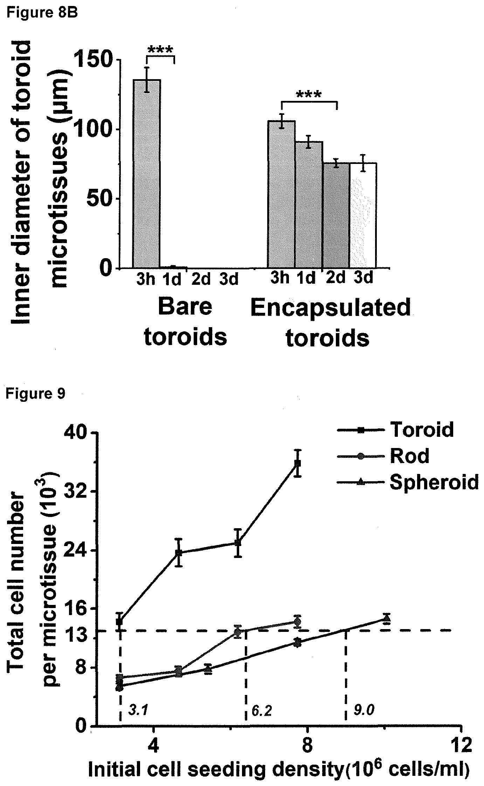

**Figure 8** shows optical images illustrating the structural changes of encapsulated and bare toroid microtissues over 3 days in culture. **Fig. 8A:** Representative optical images of a bare toroid microtissue on tissue culture plate coated with a flat layer of agarose and a toroid microtissue encapsulated in an alginate microcapsule at different time points after microtissue retrieval from the cellular assembly process. The lumen of the bare toroid microtissue closed after 1 day while that of the encapsulated microtissue persisted till day 3. The arrows indicate the surface of the alginate microcapsule. **Fig. 8B****:** Quantitative analysis of the inner diameters of bare and encapsulated microtissues (n=10 replicate samples) at different time points. Data is shown as mean ± SEM (***) denotes p < 0.005. Scale bars representing 200 µm apply to all images.
**Figure 9** shows estimated initial cell seeding density for standardization of cell number per microtissues for each geometry using the relationship between initial cell seeding density and cell number per microtissue in Fig. 2. Estimated initial cell seeding densities for toroid, rod and spheroid microtissues to have approximately 13000 cells per microtissue were 3.1, 6.2 and 9.2 million cells /mL respectively.
**Figure 10** shows measurement of microtissue dimension for calculation of their surface-to-volume ratio and volume as reported in **Fig. 3G****.** Three images in each row were taken from a single experiment during which microtissues of all three geometries were fabricated from the same batch of cells (n=3 batches of microtissues, 53 microtissues per batch). The white lines on the microtissues were added after image acquisition to indicate the positions where dimension was measured. The scale bars representing 400 µm apply to all images acquired at the same magnification.

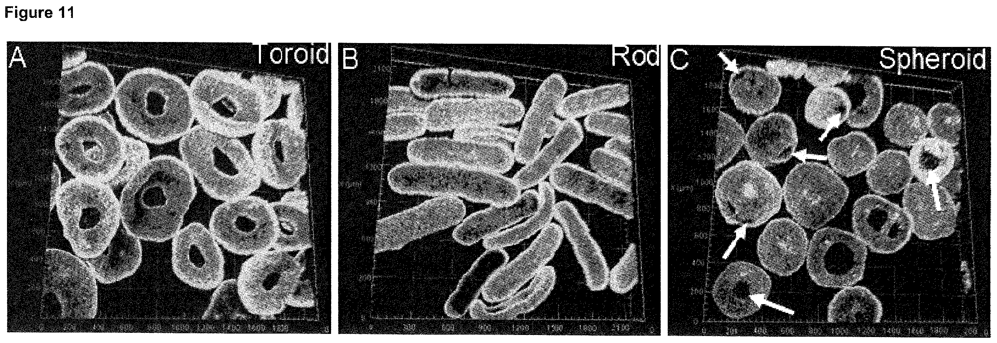

**Figure 11** shows snapshots of the 3D confocal visualization of the microtissues after live/dead staining. **Fig. 11A****:** Toroid microtissues. **Fig. 11B****:** Rod microtissues. **Fig. 11C**: Spheroid microtissues. Arrows indicated holes through which cellular debris might have been discharged from the surface of spheroid microtissues.

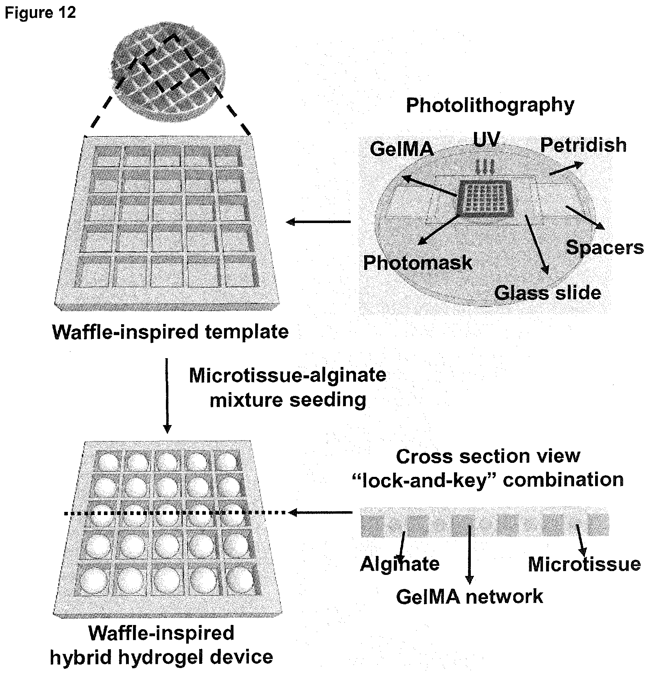

**Figure 12** shows a schematic diagram of the fabrication of waffle-inspired macrodevice comprising of multiple empty microwells for encapsulation of therapeutic microtissues that were fabricated *ex situ* prior to their addition into the macrodevice.

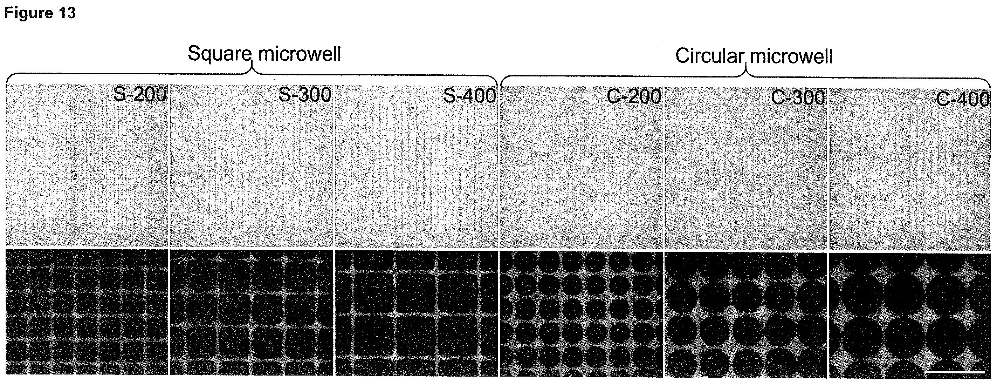

**Figure 13** shows bright field and fluorescent images of GelMA micropatterns with aligned arrays of square or circular microwells with different sizes; 200 µm, 300 µm or 400 µm well width.

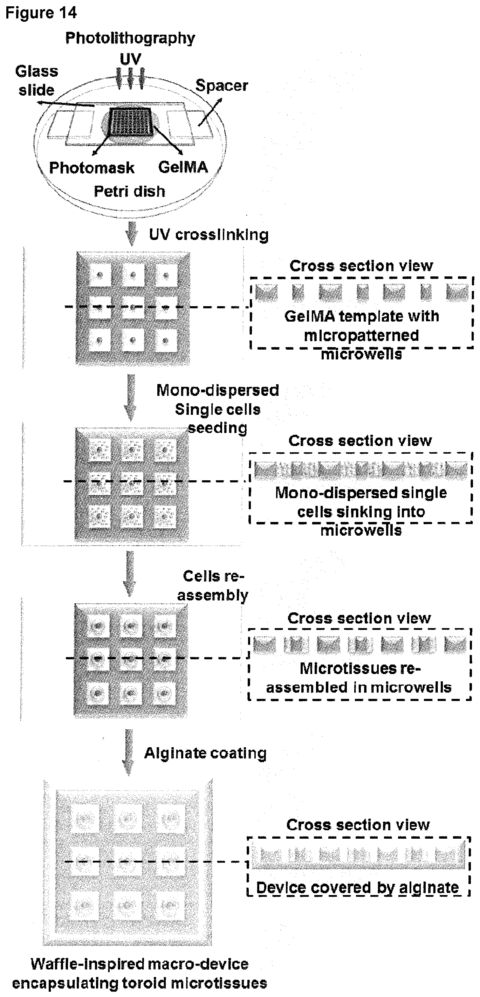

**Figure 14** shows a schematic diagram of the fabrication of waffle-inspired macrodevice comprising of multiple microwells each with a peg at its center and the *in situ* directed assembly of single cells in this device to form a toroid-shaped microtissue in each microwell.

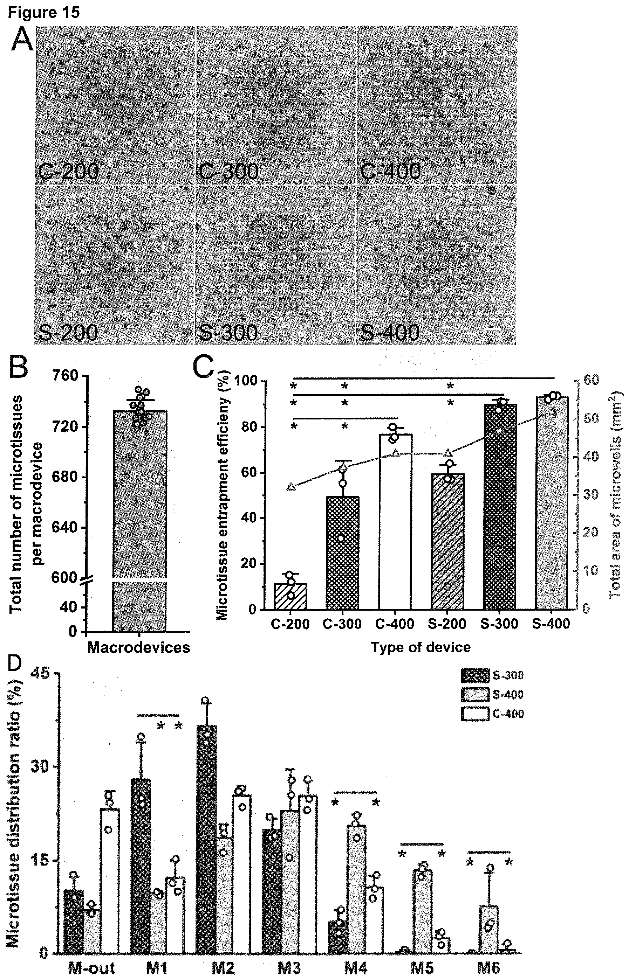

**Figure 15A-D** show microtissue distribution in macrodevices with different micropattern designs of GelMA hydrogel networks. **Fig. 15A****:** Bright filed images of macrodevices after encapsulation of microtissues. **Fig. 15B****:** Total number of encapsulated microtissue per macrodevice (1cmx1cm) (n=18 devices, 3 batches, error bar represents SD). **Fig. 15C****:** Quantitative analysis of microtissue entrapment efficiency and total area of microwells per type of macrodevice (n=1 device from N=3 independent experiment, error bar represents SD). Microtissue entrapment efficiency is defined as the ratio of the total number of microtissues entrapped in the microwells to the total number of microtissues encapsulated in the entire macrodevice. **Fig.15D****:** Quantitative analysis of microtissue distribution ratio in S-300, S-400 and C-400 macrodevices. Microtissue distribution ratio is defined as the ratio of the combined number of microtissues in all microwells of type Mn, where n is the number of microtissue(s) per microwell and ranges from 1 to 6, to the total number of microtissues encapsulated in the entire macrodevice (n=1 devices from N=3 independent experiment, error bar represents SD). (*) denotes statistical difference (p < 0.05) determined using one-way ANOVA. Scale bars: 1 mm.

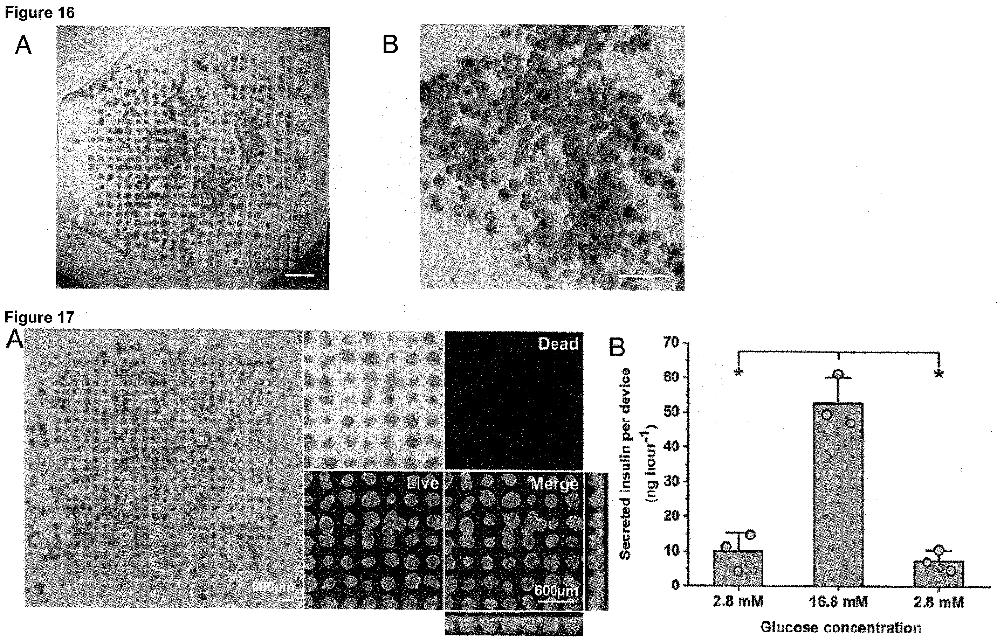

**Figure 16A-B** show bright filed images of detached microtissue-loaded device with GelMA micropattern (**Fig. 16A**) and control microtissue-loaded device without GelMA micropattern (**Fig. 16B**).
**Figure 17A-B** show characterization of viability and insulin secretion function of therapeutic microtissue in macrodevice. **Fig. 17A****:** Bright filed and fluorescent live/dead images of microtissues encapsulated in the device; **Fig. 17B****:** Static glucose stimulated insulin secretion test by consecutively subjecting the sample to three consecutive incubation in 2.8, 16.8 and 2.8 mM glucose solutions. (n=3 devices, error bar represents SD). (*) denotes statistical difference (*p* < 0.05) determined using Student's t-test.

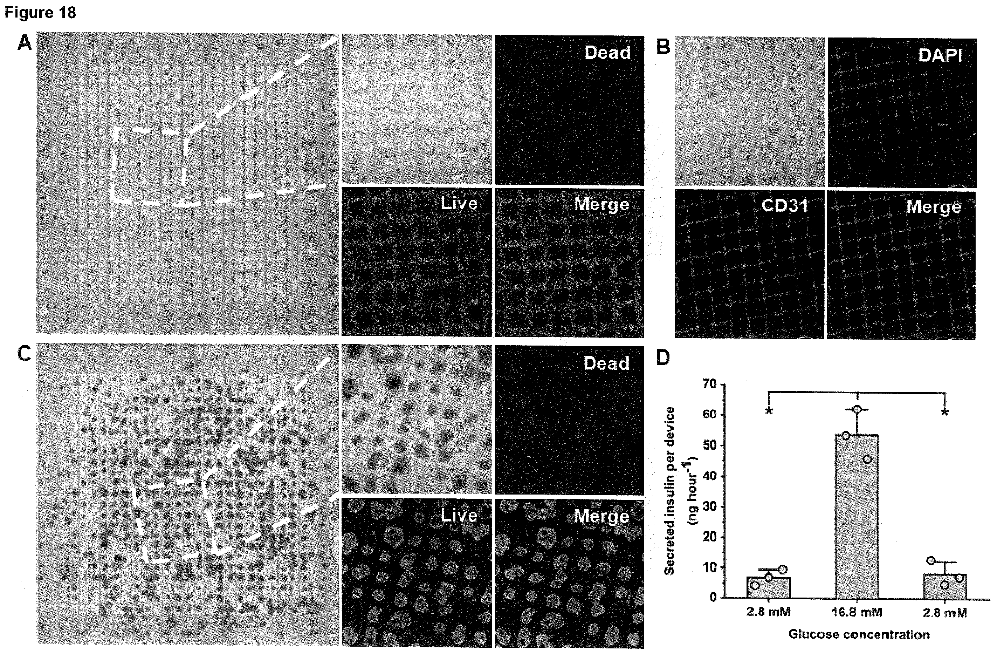

**Figure 18A-D** show functional characterizations of macrodevices containing HUVECs. **Fig. 18A****:** Bright filed and live/dead images of waffle-like macrodevice with HUVEC in its GelMA sidewall; **Fig. 18B****:** Immunofluorescent images of the device after staining of endothelial marker CD31; **Fig. 18C****:** Bright field and fluorescent live/dead images of macrodevice device co-encapsulating INS-1E microtissues in microwells and HUVECs in GelMA sidewall; **Fig. 18D****:** Static glucose-stimulated insulin secretion test by subjecting the device encapsulating both therapeutic microtissues and HUVECs to three consecutive incubation in 2.8mM,16.8mM and 2.8 mM glucose solutions. (n=3 devices, error bar represents SD). (*) denotes statistical difference (p < 0.05) determined using student's t-test.

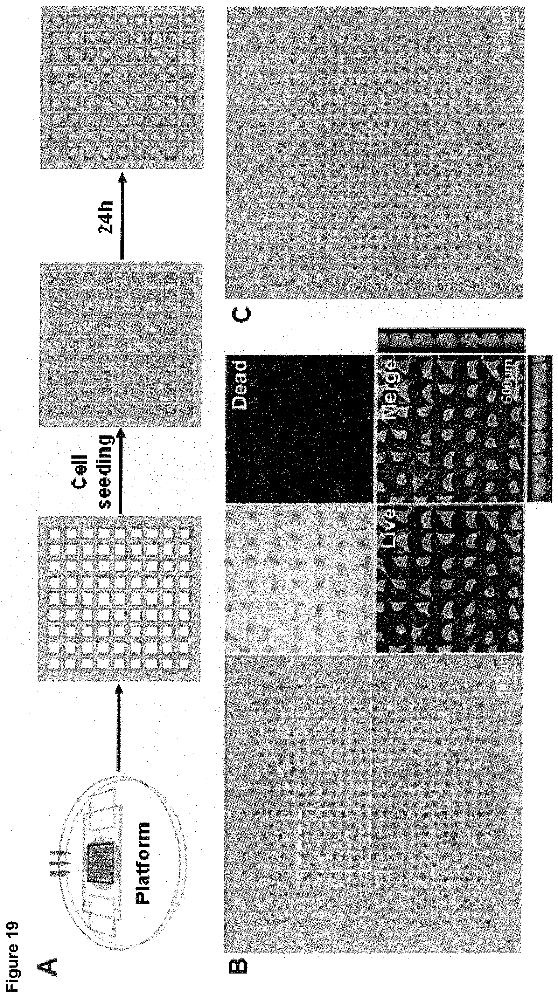

**Figure 19A-C** show the formation of therapeutic microtissues *in situ* by guided assembly of mono-dispersed cells on a waffle-like GelMA hydrogel micropattern. **Fig. 19A****:** Schematic fabrication of assembly of monodispersed cells for *in situ* formation of therapeutic microtissues on micropattern S-300; **Fig. 19B****:** Bright field and live/dead images of the microtissues formed on micropattern from 0.8 million of seeded cells; **Fig. 19C****:** Bright field image of microtissues formed on micropattern from 0.4 million of seeded cells.

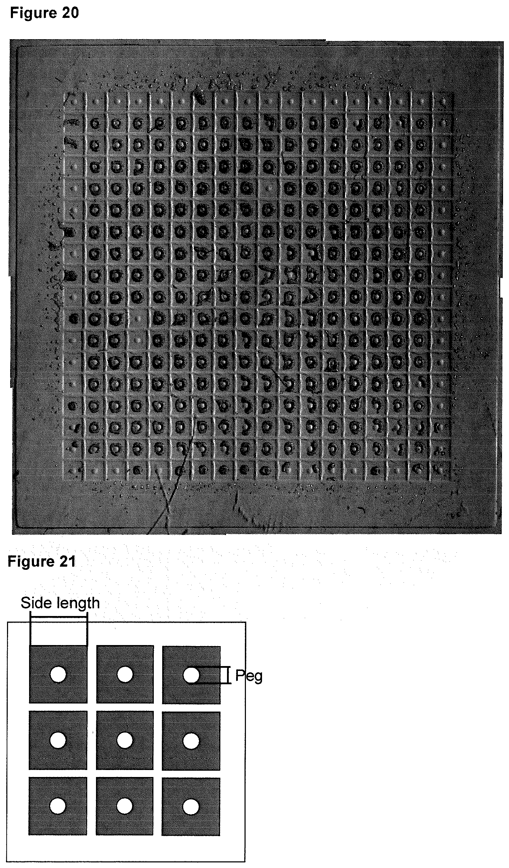

**Figure 20** shows a bright field image of toroid microtissues formed by guided assembly of 0.4 million seeded cells on GelMA hydrogel micropattern with a peg at the center of each microwell. The micropattern used in this experiment was S-400 which consisted of array of square microwells with 400 µm side width dimension and the diameter of the peg-like structure in each microwell was 100 µm.
**Figure 21** is a schematic diagram of a waffle-inspired macroencapsulation device similar to that shown in Figure 20, with square microwells (clear region) and circular pegs (dark circles).

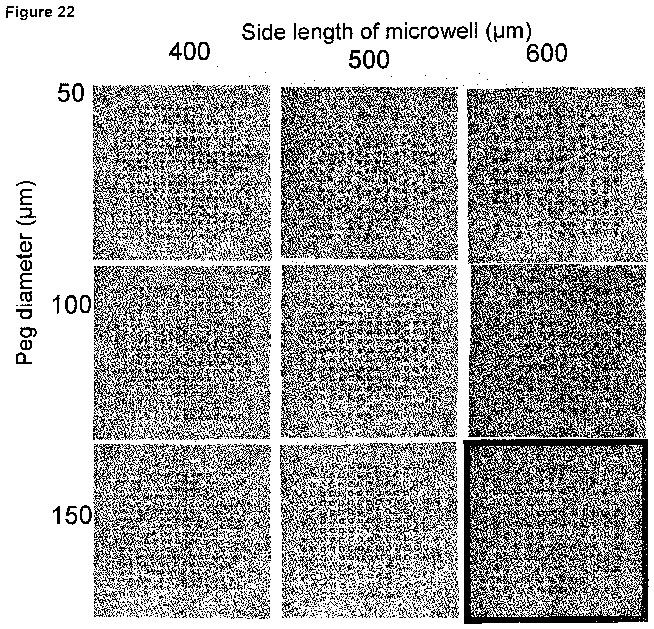

**Figure 22** shows bright field images of toroid-loaded waffle-inspired microencapsulation device to compare the effects of peg diameter and microwell side width dimension on toroid morphology.

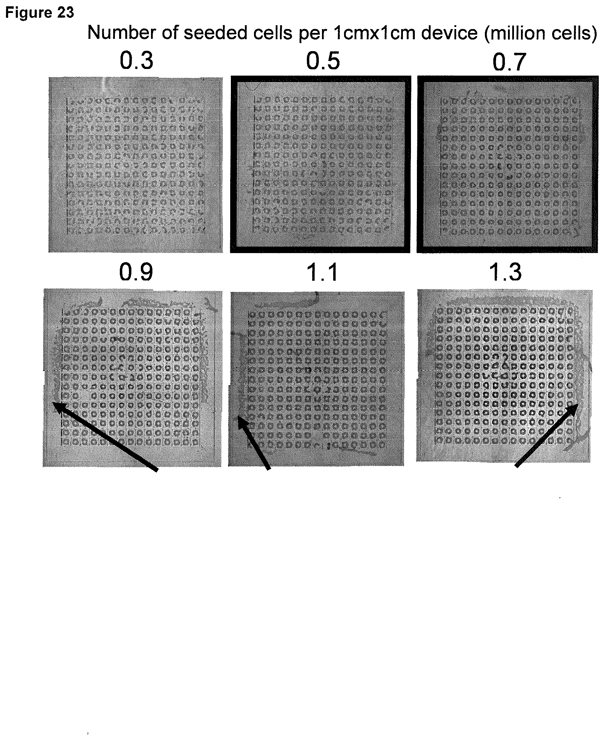

**Figure 23** shows bright field images of toroid-loaded waffle-inspired macroencapsulation device to compare the effects of initial cell loading on *in situ* formation of toroid microtissue. Arrows indicate cell sheet formation on the edge of the device due to excessive initial cell loading.

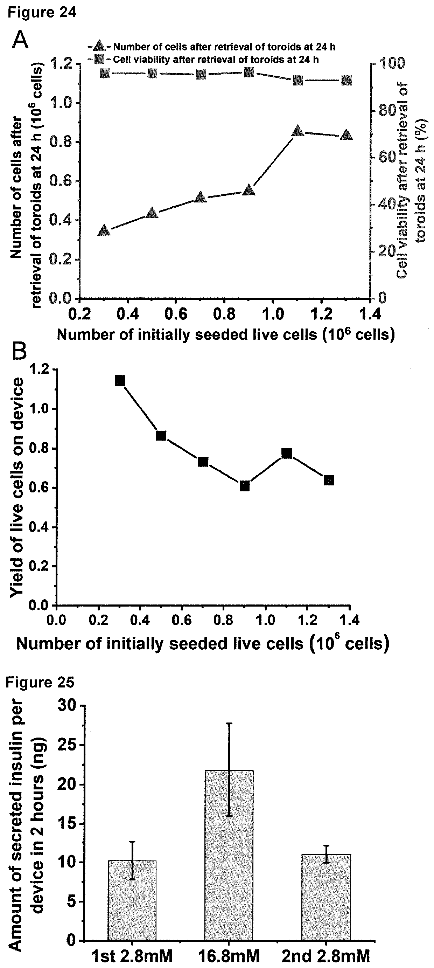

**Figure 24** shows graphs illustrating the effect of initial cell loading on cell viability and yield at 24 hours post-seeding. **Fig. 24A****:** Cell viability was maintained at above 90% at all initial cell loadings. **Fig. 24B****:** Live cell yield was highest in the lower range of initial cell loadings.
**Figure 25** shows insulin secretion from toroid microtissues in waffle-like macrodevice in response to static glucose stimulation. Microtissue-containing waffle-like macrodevices were consecutively incubated in low glucose (2.8 mM) and high glucose (16.8 mM) followed by low glucose (2.8 mM) for 2 hours at each concentration. A higher average amount of insulin was secreted in response to the higher glucose level. Data is shown as mean ± SEM (n=3 devices, error bar represents SD).

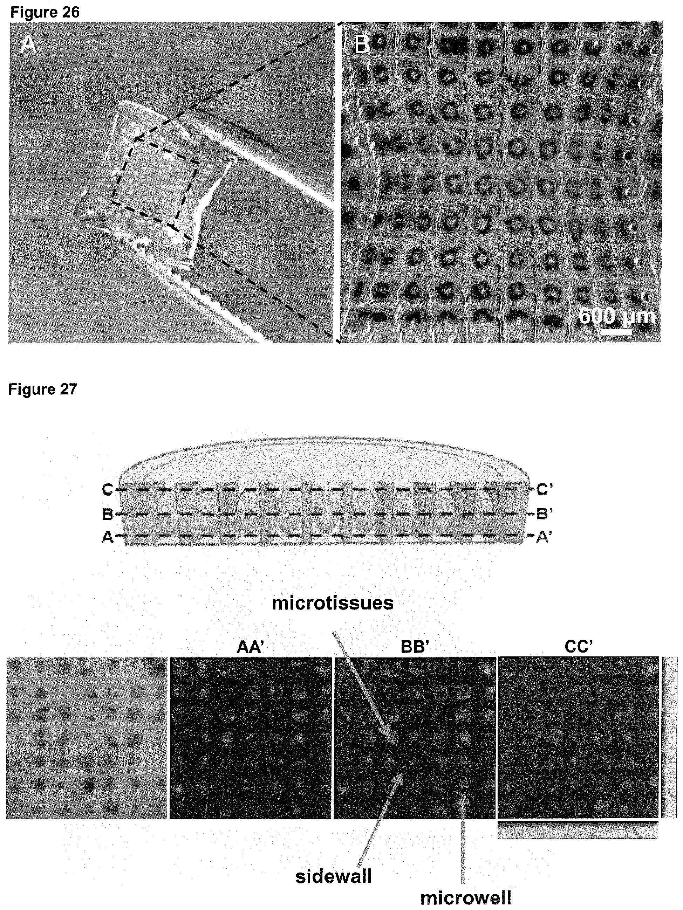

**Figure 26A-B** show photographs of a waffle-inspired macrodevice containing toroid microtissues. **Fig. 26A****:** The macrodevice had strong mechanical property to sustain handling with tweezers. **Fig. 26B****:** Minimal deformation of the waffle arrangement was observed under magnification.
**Figure 27** shows a schematic and bright field image and confocal cross sections (AA', BB', CC') at three different depths of a waffle-inspired macrodevice. "Lock" or "interlocking" component of GelMA hydrogel network was visualized by mixing GelMA with green fluorescent microbeads (arrow marked as "side wall") while the second interlocking component of alginate-microtissue mixture was visualized by adding red fluorescent microbeads to the alginate (arrow marked as "microwell"). INS-1E microtissues in alginate "key" component was stained with blue Hoechst dye (arrows marked as "microtissues"). Appearance of all three signals in each confocal plane illustrated the interlocking nature of the two modular components.

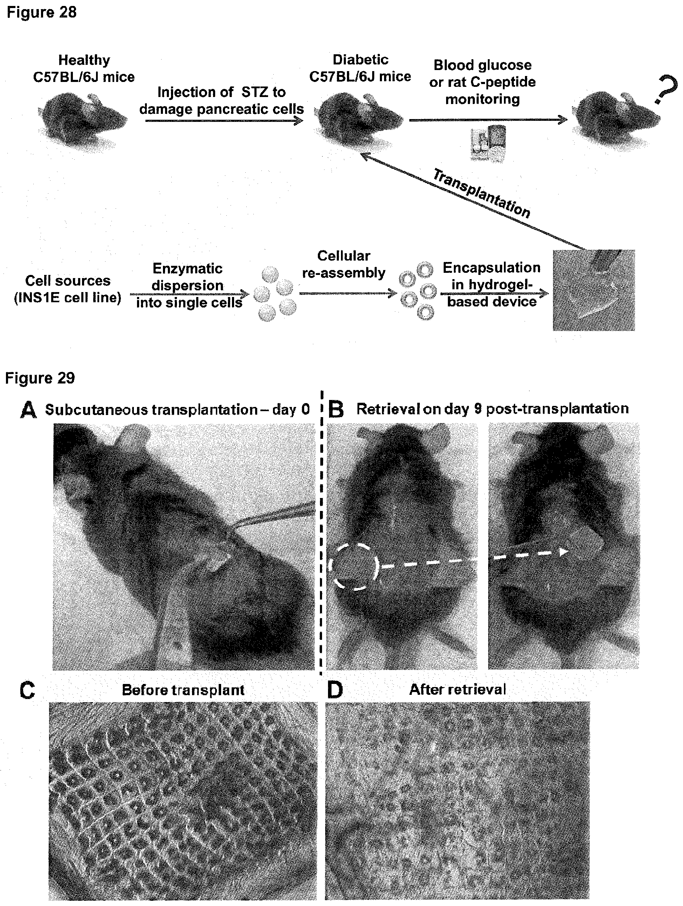

**Figure 28** shows a schematic diagram of the procedures for generation of a chemically induced diabetic mouse model, fabrication of the waffle-inspired macrodevice and its transplantation into a diabetic mouse.
**Figure 29** shows photographs of the macrodevice before transplantation (**Fig. 25A**) and upon retrieval after 9 days post-transplanation from a diabetic mouse (**Fig. 25B**). The macrodevice retained its structural and mechanical stability to sustain handling with tweezers during device retrieval. In brightfield images under magnification (**Fig. 25C-D**), minimal deformation of the waffle arrangement is observed in the retrieved device (**Fig. 25D**) compared to pre-transplant device (**Fig. 25C**).

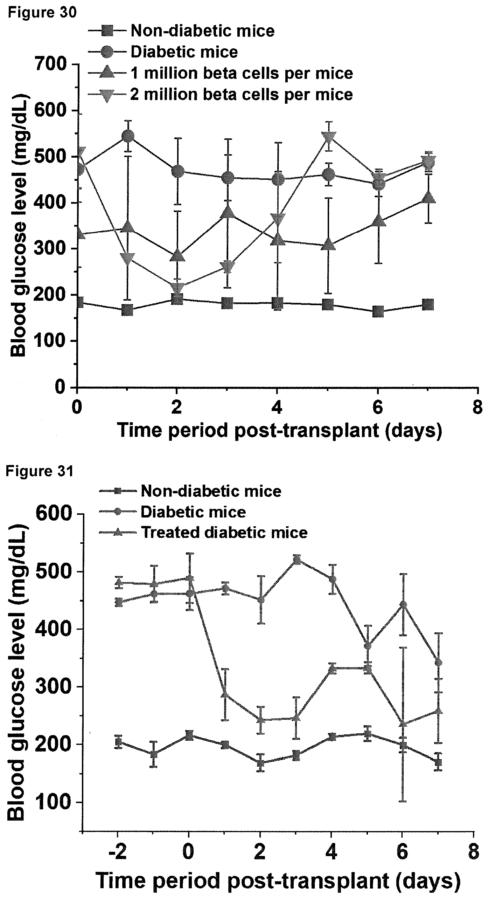

**Figure 30** shows a graph of daily non-fasting blood glucose level against time for non-diabetic mice, control STZ-induced diabetic mice, STZ-induced diabetic mice subcutaneously transplanted with waffle-like macrodevices containing toroid microtissue at therapeutic doses of 1 million or 2 million beta cells per mouse. Diabetic mice receiving the toroid-loaded waffle-like macrodevice at a therapeutic dose of 2 million cells per mouse lowered blood glucose level of diabetic mice just 1 day post-transplantation.
**Figure 31** shows a graph of daily non-fasting blood glucose level against time for non-diabetic mice, diabetic mice, and control STZ-induced diabetic mice, treated STZ-induced diabetic mice intraperitoneally transplanted with toroid microtissues encapsulated in alginate microcapsules. Diabetic mice treated with microencapsulated toroid microtissues could lower blood glucose level of diabetic mice to below 300 mg/dL just 1 day post-transplantation

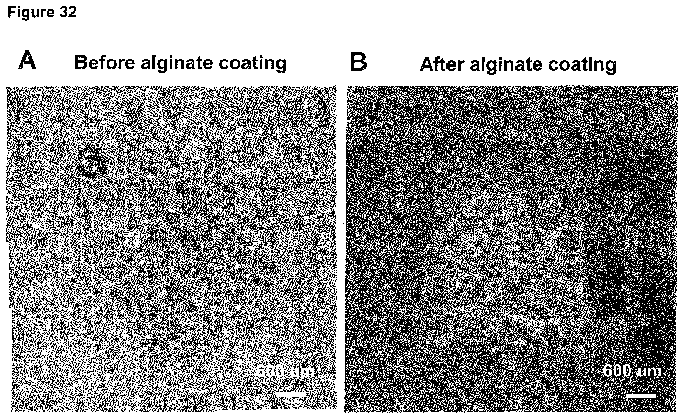

**Figure 32** shows brightfield images of waffle-like macrodevice encapsulating primary rat islets. **Fig. 32A****:** Alginate-islet mixture was dispensed on GelMA hydrogel network on a glass slide illustrating the ability of the micropatterned design to distribute the therapeutic microtissues homogeneously by entrapping one microtissue per microwell. **Fig 32B****:** After further external coating with a barium-crosslinked alginate hydrogel layer, the macrodevice still maintained the homogenous islet distribution after its detachment from the glass slide.
Bibliographic references mentioned in the present specification are for convenience listed in the form of a list of references and added at the end of the examples.

### DETAILED DESCRIPTION OF THE INVENTION

### Definitions

Certain terms employed in the specification, examples and appended claims are collected here for convenience.

The terms "a" and "an" are used herein to refer to one or to more than one (i.e. at least one) of the grammatical object of the article.

As used herein, the term "comprising" or "including" is to be interpreted as specifying the presence of the stated features, integers, steps or components as referred to, but does not preclude the presence or addition of one or more features, integers, steps or components, or groups thereof. However, in context with the present invention, the term "comprising" or "including" also includes "consisting of". The variations of the word "comprising", such as "comprise" and "comprises", and "including", such as "include" and "includes", have correspondingly varied meanings.

The term "component" as used herein is intended to describe each of the crosslinked hydrogels that define an array of microwells and the cross-linked immuno-isolatory hydrogel comprising microtissues. First and second component may also be used to define the respective hydrogels.

The term "composition" is defined as a combination of different materials, such as polymer and cells. The composition may comprise a planar matrix (also called a "waffle-inspired" structure), made of a transparent or translucent polymer. The cells may be arranged in a packing arrangement, such as a square packing arrangement consisting of columns and rows. The cells may be spaced at regular intervals. The cells may form clusters, such as rods, spheroids or toroid-shaped building units.

The term "cluster" or "cell cluster" is defined as a group of cells that are clumped together or self-assembled into a microtissue.

The term "toroid-shaped", "toroidal" or "toroid microtissue" used herein to describe a ring- or donut-shaped tissue having an open lumen.

The term "lock", "locking" or "interlock", "interlocking" as used herein is intended to describe the interdigitating of a) an interconnected network of cross-linked hydrogel, which serves as dividing sidewalls separating evenly spaced microwells, wherein each microwell in the array comprises at least one side wall and is configured to encapsulate a single therapeutic microtissue and b) therapeutic microtissues in an immuno-isolatory hydrogel that, when cross-linked, are entrapped in the microwells, and wherein said immuno-isolatory hydrogel component of b) interlocks with the interconnected hydrogel network component of a) to form essentially a unitary macrodevice. More simply it is the cross-linking of the immuno-isolatory hydrogel within, and over, the wells of the macrodevice, for example as shown in the schematic in Figures 12 and 14.

The term "polymer" or "biopolymer" is defined as a substance with repeated molecular units. The polymer may be a biocompatible polymer, selected from the group comprising polysaccharide (e.g. agarose), polyphosphazene, poly(acrylic acids), poly(methacrylic acids), copolymers of acrylic acid and methacrylic acid , poly(alkylene oxidese), poly(vinyl acetate), polyvinylpyrrolidone (PVP), and copolymers and blends thereof. The polymer may be polyacrylamide or a blend comprising polyacrylamide. The polymer may be a flexible polymer that is also mechanically and structurally stable and suitable for transplantation or implantation (e.g. subcutaneous transplantation or implantation). The polymer may or may not be biodegradable.

The term "mammalian cell" is defined as any cell derived from a mammalian subject. The cell may be secretory (e.g. pancreatic cells), structural (e.g. mesenchymal or epithelial cells), or metabolic. The cell may be selected from the group including but not limited to any of the following cell types: islets of Langerhans, bone marrow mononuclear cells, mesenchymal stem cells, mobilized peripheral blood mononuclear cells, endothelial progenitor cells, follicular cells, Leydig cells, ovarian cells, neural stem cells, human embryonic stem cells, pluripotent stem cells, skeletal myoblasts, cardiomyoblasts, etc. The cell may be xenogeneic, autologous or allogeneic. The cell can be a primary cell obtained directly from a mammalian subject. The cell may also be a cell derived from the culture and expansion of a cell obtained from a subject. The cell may be a stem cell. Immortalized cells (i.e. cell lines) are also included within this definition. In some embodiments, the cell has been genetically engineered to express a recombinant protein and/or nucleic acid.

The term "subject" is herein defined as vertebrate, particularly mammal, more particularly human. For purposes of research, the subject may particularly be at least one animal model, e.g., a mouse, rat and the like. In particular, for treatment or prophylaxis of a disease, such as type 1 diabetes, the subject may be a human.

The term 'treatment', as used in the context of the invention refers to prophylactic, ameliorating, therapeutic or curative treatment.

For purposes of brevity and clarity, descriptions of embodiments of the present disclosure are directed to hydrogel-encapsulated microtissues and a hybrid hydrogel macrodevice for therapeutic implantation, in accordance with the Examples. While aspects of the present disclosure will be described in conjunction with the embodiments provided herein, it will be understood that they are not intended to limit the present disclosure to these embodiments.

The present disclosure provides a waffle-inspired hybrid hydrogel macrodevice that may be utilized to facilitate enhanced performance of cell-based therapeutics by facilitating distribution of encapsulated microtissues and formation of interconnected vasculature to improve microtissue performance in a wide range of applications.

According to a first aspect of the disclosure, there is a planar biocompatible hydrogel-based macrodevice comprising an array of microwells and a therapeutic microtissue within each of a plurality of said microwells, wherein;
a) said macrodevice comprises an interconnected network of cross-linked hydrogel, which serves as dividing sidewalls separating evenly spaced microwells, wherein each microwell in the array comprises at least one side wall and is configured to encapsulate a single therapeutic microtissue; and
b) said macrodevice comprises therapeutic microtissues in an immuno-isolatory hydrogel that, when cross-linked, are entrapped in the microwells, and
wherein said immuno-isolatory hydrogel of b) interlocks with the interconnected hydrogel network of a).

It would be understood that there are several biopolymers that could be used to generate the macrodevice. The polymer may be a biocompatible polymer, selected from the group comprising polysaccharide (e.g. agarose), polyphosphazene, poly(acrylic acids), poly(methacrylic acids), copolymers of acrylic acid and methacrylic acid , poly(alkylene oxidese), poly(vinyl acetate), polyvinylpyrrolidone (PVP), collagen, elastin, polyethylene glycol, alginate, hyaluronic acid or their derivatives and copolymers and blends thereof. The polymer may be polyacrylamide or a blend comprising polyacrylamide. The polymer may be a flexible polymer that is also mechanically and structurally stable and suitable for transplantation or implantation (e.g. subcutaneous transplantation or implantation). In some embodiments it is preferred that the hydrogel is gelatin methacryloyl (GelMA).

In some embodiments, the at least one side wall of each of a plurality of said microwells further comprise vascular endothelial cells. The endothelial cells are embedded within the walls to promote vascularization and support for the microtissues within each well.

The planar biocompatible hydrogel-based macrodevice further comprises a coating of an immuno-isolatory hydrogel, such as an alginate hydrogel, that encapsulates the macrodevice. The extra coating over the device provides additional structural support or mechanical strength to an implanted macrodevice.

In some embodiments the microwells comprise a peg arranged to guide the formation of a toroid-shaped microtissue. Non-limiting Examples are shown in Figures 17 to 19. In some embodiments the peg has a diameter in the range of 50 µm to 200 µm, preferably a diameter of about 100 µm. In some embodiments of the planar biocompatible hydrogel-based macrodevice, the peg has a diameter of 100 µm and the width dimension of each of said microwells is 500 µm.

In some embodiments the microwells are square or circular in the horizontal plane, such as shown in Figure 11.

The dimensions of the microwells are herein expressed in terms of width dimension, which means, for example, that for a square well, the width dimension is the length of one side in cross-section; for a circular microwell the width dimension is the diameter of the circle cross-section. Essentially, it is the distance from one side of the well to the other.

In some embodiments the width dimension of each of said microwells is in the range of 100 µm to 1000 µm, preferably 300 µm to 500 µm, more preferably about 500 µm. Preferably, the peg has a width dimension of 100 µm and the width dimension of each of said microwells is 500 µm.

In some embodiments the microwell side walls have a height in the range 100 µm to 1000 µm, preferably about 300 µm. The height or depth of the wells may vary depending on the nature of the implant, where it is implanted and the type of microtissues used. 3D-printed macrodevices of the disclosure may have deeper wells.

The size of the biocompatible hydrogel-based macrodevice is not intended to be limited other than by practical constraints determined by the particular application. Examples disclosed herein show that macrodevices comprising 144 wells, 324 wells, 529 wells and 1024 wells may be used for seeding with microtissues (Figure 12). In some embodiments the planar biocompatible hydrogel-based macrodevice comprises at least 100 microwells, at least 300 microwells, at least 500 microwells or at least 1000 microwells.

In some embodiments the therapeutic microtissue is rod-shaped, spherical, or toroid-shaped, preferably toroid-shaped.

In some embodiments the microtissues comprise secretory, structural or metabolic cells. In some embodiments the microtissues comprise cells selected from the group comprising insulin-secreting islets of Langerhans cells [Song, S. and Roy, S. Biotechnol Bioeng. 113(7): 1381-1402 (2016)], albumin-secreting hepatocytes [Glicklis R, et al., Biotechnol Bioeng. 86(6): 672-680 (2004); Gionet-Gonzales, MA and Leach, JK. Biomedical materials. 13(3): 034109 (2018)], bone marrow mononuclear cells, mesenchymal stem cells, mobilized peripheral blood mononuclear cells, endothelial progenitor cells, follicular cells, Leydig cells, ovarian cells, neural stem cells, human embryonic stem cells, pluripotent stem cells, skeletal myoblasts and cardiomyoblasts or genetically engineered cells for growth hormone deficiency or haemophilia [Gao K, et al., Stem cell research & therapy. 10(1): 34 (2019)]. In some embodiments the microtissues secrete a therapeutically effective substance, such as a hormone or protein.

In some embodiments the cross-linked hydrogel in a) is preferably GelMA and/or the immuno-isolatory hydrogel in b) is preferably alginate.

In some embodiments each of the said therapeutic microtissues:
a) was added to the said microwells, or
b) was generated within the said microwells from a cell suspension.

In some embodiments the cell suspension comprises about 0.5 to 1.5 million cells per cm² of macrodevice.

According to a second aspect of the present disclosure, there is a method of manufacturing a planar biocompatible hydrogel-based macrodevice, comprising the steps;
a) hydrogel prepolymer is dispensed onto a surface;
   i) a photomask with an array of holes or transparent features of specified geometry and dimensions is placed over the dispensed mixture, and
   ii) the mixture is exposed to UV light or solution containing cross-linker to crosslink an exposed portion of the hydrogel prepolymer, to form a micropatterned network of sidewalls, and
   iii) the crosslinked hydrogel pattern is rinsed to remove non-crosslinked hydrogel residue, leaving behind microwells; or
b) hydrogel prepolymer is dispensed onto a surface using 3D printing and cross-linked to form a micropatterned network of sidewalls defining microwells;
c) adding a mixture of microtissues and cross-linkable immuno-isolating hydrogel onto the crosslinked hydrogel micropattern of a) or b), and crosslinking the immunoisolating hydrogel; or
d) adding a suspension of cells to the microwells and culturing the cells under suitable conditions until the cells aggregate into desired microtissue shape depending on the chosen cross-linked hydrogel micropattern, and
   (i) adding cross-linkable immuno-isolating hydrogel onto the cross-linked hydrogel micropattern, and
   (ii) crosslinking the immuno-isolating hydrogel,
wherein the planar biocompatible hydrogel-based macrodevice comprises a single microtissue per microwell.

The method further comprises a step e) encapsulating the macrodevice and microtissues in a biocompatible hydrogel layer, such as an alginate hydrogel layer. This further encapsulation of the macrodevice provides extra support and protection of the macrodevice when used, for example, as an implant.

In some embodiments the cells are seeded onto the macrodevice at about 0.3 to 1.5 million cells per cm² of macrodevice.

In some embodiments the microtissue and/or cell suspension are selected from the group comprising islets of Langerhans cells, hepatocytes, bone marrow mononuclear cells, mesenchymal stem cells, mobilized peripheral blood mononuclear cells, endothelial progenitor cells, follicular cells, Leydig cells, ovarian cells, neural stem cells, human embryonic stem cells, pluripotent stem cells, induced pluripotent stem cells (iPSCs), skeletal myoblasts, cardiomyoblasts and genetically engineered cells for growth hormone deficiency or haemophilia.

In some embodiments, in step a) or b) a mixture of hydrogel prepolymer and vascular endothelial cells is dispensed onto said surface and wherein said vascular endothelial cells are embedded in the microwell side walls.

In some embodiments the hydrogel prepolymer in step a) or b) is GelMA and/or the cross-linkable immuno-isolating hydrogel in step c) or d)i) is alginate.

According to a third aspect of the present disclosure, there is a composition comprising cells for implantation, wherein the composition comprises a plurality of biocompatible hydrogel-based microcapsules each having a toroid-shaped microtissue encapsulated therein, wherein said microtissue secretes a therapeutically effective substance, such as a hormone or protein.

In some embodiments the hydrogel is a cross-linkable immuno-isolating hydrogel.

In some embodiments the hydrogel is preferably alginate.

In some embodiments the microtissue comprises cells selected from the group comprising islets of Langerhans cells, hepatocytes, bone marrow mononuclear cells, mesenchymal stem cells, mobilized peripheral blood mononuclear cells, endothelial progenitor cells, follicular cells, Leydig cells, ovarian cells, neural stem cells, human embryonic stem cells, pluripotent stem cells, induced pluripotent stem cells (iPSCs), skeletal myoblasts, cardiomyoblasts and genetically engineered cells for growth hormone deficiency or haemophilia.

According to a fourth aspect of the present disclosure, there is a method of treatment comprising implanting into a subject in need of such treatment a planar biocompatible hydrogel-based macrodevice of any aspect of the disclosure or a composition of any aspect of the disclosure.

In some embodiments the planar biocompatible hydrogel-based macrodevice or the composition comprises microtissues that secrete insulin to treat diabetes.

In some embodiments the microtissues comprise islet cells.

According to a fourth aspect of the present disclosure, there is use of a planar biocompatible hydrogel-based macrodevice of any aspect of the disclosure or a composition of any aspect of the disclosure as an implant for treating a subject.

According to a fifth aspect of the present disclosure, there is kit comprising a planar biocompatible hydrogel-based macrodevice of any aspect of the disclosure or a composition of any aspect of the disclosure.

In some embodiments the kit is used to treat a subject in need thereof. For example, a subject may need treatment for diabetes and said kit comprises a macrodevice or composition according to any aspect of the disclosure comprising insulin-secreting microtissues.

### EXAMPLES

### EXAMPLE 1: Methods for fabrication, characterization of microtissues and their encapsulation in alginate microcapsules

### 1.1. Fabrication of micromolded agarose hydrogels

As illustrated in **Fig.1A****,** sSilicone templates 3D Petri Dish^{®} (Sigma-Aldrich, USA) were utilized to prepare micropatterned agarose hydrogels consisting of multiple recesses with desired geometries [Napolitano, A.P. et al., Biotechniques, 43: 494, 496-500 (2007)]. Ultrapure^{©} Agarose (Invitrogen, Carlsbad, USA) powder was autoclaved and dissolved by heating in sterile Mili-Q water to form a 2.3% (w/w) solution which was subsequently added into each silicon template and allowed to solidify at room temperature for 25 min. Afterwards, solidified micromolded agarose hydrogels were detached from the silicone template using a spatula and transferred to a sterilized 6-well tissue culture plate. Each micromolded agarose hydrogel was equilibrated with 3 mL of culture medium overnight before cell seeding. Toroid-fabricating recesses, arranged in a 6 × 6 array per agarose gel, were each 750 µm deep and 1400 µm wide. Their central pegs and surrounding circular tracks had diameters of 600 µm and 400 µm respectively. Rod-fabricating recesses, arranged in a 15 × 6 array per agarose gel, were each 2200 µm long, 400 µm wide and 750 µm deep. Sphere-fabricating recesses, arranged in a 9 × 9 array per agarose gel, were each 800 µm deep with a round bottom of 800-µm diameter.

### 1.2. Cell culture

Rat insulinoma cell line (INS-1E) was a generous gift from Dr. Yusuf Ali (Lee Kong Chian School of Medicine, Nanyang Technological University, Singapore) with permission from Professor Claes B. Wollheim (Lund University, Sweden). INS-1E cells at passages of 96-110 were cultured according to a published protocol [Napolitano, A.P. et al., Tissue Eng., 13: 2087-2094 (2007)]. Cells were cultured in RPMI complete medium which consisted of RPMI-1640 medium (Hyclone, USA) supplemented with 10% heatinactivated fetal bovine serum (Gibco Laboratories, USA), 2 mM L-glutamine, 10 mM HEPES, 1 mM sodium pyruvate, 100 U/mL penicillin, 100 µg/mL streptomycin and 50 µM 2-mercaptoethanol. Cells were maintained in a humidified atmosphere containing 95% air and 5% CO₂ at 37 °C. At a confluency of about 70%, cells were detached after treatment with 0.25% trypsin-EDTA (Gibco Laboratories, USA) for 2 min at 37 °C. Subculture was performed at a 1:4 split ratio with a plating density of 2×10⁵ cells/cm². Culture medium was replaced every 3 days.

### 1.3. Fabrication of islet-like microtissues

Seeding of INS-1E cells onto micromolded agarose hydrogel was carried out as previously described [Dahl, U. et al., Development, 122: 2895-2902 (1996)]. Typically, a 175 µL volume of single cell suspension with desired density was pipetted into each micromolded agarose hydrogel. Cells were allowed to sink to the bottom of the recesses for 30 min before 2.5 mL of the culture medium was added to the micromolded agarose hydrogel. Typically, after 55 h from initial seeding of monodispersed INS-1E cells, microtissues were retrieved using pipette loaded with large orifice filter tips (VWR, USA) and rinsed 3 times with RPMI complete medium and kept in the same medium prior to further characterization. Microtissues were analysed immediately after retrieval for number of cells per microtissues, percentage of death cells, metabolic activity and live/dead staining. Retrieved microtissues were cultured in RPMI complete medium and kept in an incubator at 37oC, 5% CO₂ for 2.5 hour prior to GSIS assay.

### 1.4. Cell counting and trypan blue exclusion assay of dissociated microtissues

The number of cells per microtissue and the viability of the microtissues were characterized with trypan blue exclusion assay (Life Technologies, USA). A volume of 200 µL of the culture medium containing 10 microtissues was transferred into a 1.5 mL Eppendorf tube. After the microtissues settled into the bottom of the Eppendorf tube, 185 µL of the culture medium was removed, and the microtissues were rinsed with 200 µL of phosphate-buffered saline (PBS) (Hyclone, USA) twice. Subsequently, each tube was added with 40 µL of 0.25% trypsin-EDTA and incubated at room temperature for 10 min. Afterwards, 95 µL of the culture medium was added into the tubes to inactivate the trypsin, and the microtissues was then dissociated into single cell suspension by gentle pipetting. The cell suspension was stained with trypan blue at a 1:1 volume ratio and counted with a COUNTESS^{™} II automated cell counter (Thermal Fisher Scientific, IL, USA) to determine the cell density and the percentage of dead cells. The percentage of dead cells was also calculated as the ratio of the number of dead cells to the number of cells in the suspension.

### 1.5. Live/dead fluorescent staining of microtissues

The viability of intact microtissues was characterized using LIVE/DEAD^{™} cell viability kit (Thermo Fisher, USA). Several microtissues were transferred into a 24-well plate. Excess medium was removed and the microtissues were rinsed with 2 mL of PBS three times. After 1 mL of PBS containing 1.6 µM of calcein-AM and 13 µM of ethidium homodimer-1 were added into each well, the microtissues were incubated at 37 °C for 45 min. During this incubation, microtissues were stirred once by gentle pipetting to achieve homogenous staining. Microtissues were rinsed with 2 mL of PBS three times and visualized using a confocal microscope (ZEISS LSM 800 with Airyscan, Carl Zeiss, Germany) with the excitation wavelengths of 488 nm and 543 nm for imaging of live and dead cells respectively. The images of stained microtissues were captured at different depths with the slice interval of 1 µm for a total thickness of approximately 200 µm.

### 1.6. Evaluating metabolic activity of microtissues

The metabolic activity of microtissues was characterized using WST-1 proliferation assay (Abcam, USA). A volume of 200 µL of the culture medium containing 8 microtissues was transferred into a 96-well non-treated tissue culture plate. After 130 µL of the culture medium was removed, a 150 µL volume of a mixture containing the culture medium and WST-1 reagent at a 6.5:1 volume ratio was added into each well. The plate was incubated at 37 °C for 3 h. Afterwards, 110 µL of the supernatant was transferred to another 96-well plate and the sample absorbance at 450 nm was measured using a microplate reader (SPECTRAMAX^{®} M5, Molecular Devices, USA).

### 1.7. Characterizing surface-to-volume ratio of microtissues

The dimensions of microtissues were measured from the optical images acquired with an inverted phase contrast microscope (CKX, Olympus, Japan). Toroid, rod and spheroid microtissues were approximated to the corresponding ideal shapes of torus, cylinder and sphere respectively. The volume and surface area of toroid microtissue were calculated as *V_{T}* =0.25π² × (*a*+*b*) × (*b*-*a*)² and *S_{T}*= π² × (*b*²-*a*²) respectively, where *b* is the outer radius and a is the inner radius of the torus. The volume and surface area of rod microtissue were calculated as *V_{R} =*π*r*²*L* and *S_{R}*=2π*r* × (*r*+*L*) respectively, where r is the radius and L is the length of the cylinder. The volume and surface area of spheroid microtissue were calculated as *V_{S} =* (4/3)π × *r*³ and S_{S}=4π × *r*² respectively, where r is the radius of the sphere.

### 1.8. Static glucose-stimulated glucose secretion

A volume of 200 µL of the culture medium containing 8 microtissues were transferred into a 96-well plate. After 130 µL of the culture medium was removed, microtissues were rinsed with 250 µL of glucose-free RPMI-1640 complete medium twice to remove residual insulin. The microtissues were preincubated with the glucose-free medium for 1.5 h at 37 °C. After the microtissues were washed twice with Krebs Ringer buffer HEPES (KRBH) (135 mM NaCl, 5 mM NaHCO₃, 0.5 mM NaH₂PO₄, 3.6 mM KCI, 1.5 mM CaCl₂, 0.5 mM MgCl₂, and 10 mM HEPES, pH 7.4. BSA (0.1%)), the *in vitro* insulin secretion was evaluated by three consecutive incubations of the microtissues in 200 µL of KRBH containing 2.8 mM and then 16.8 mM glucose followed by 2.8 mM glucose at 37 °C for 1 h per incubation. The residual insulin was removed by rinsing the samples twice with glucose-free KRBH buffer between the two incubations. At the end of each incubation, 30 µL of the supernatant was removed and stored at -20 °C for subsequent analysis. The insulin concentrations were measured by Ultrasensitive Insulin ELISA (ALPCO Diagnostics, USA) and normalized by DNA content, which were quantified using CyQUANT^{®} Cell Proliferation Assay (Invitrogen, USA). Briefly, microtissue samples collected after the third incubation with glucose solution were frozen at -80 °C overnight. After thawing, each microtissue sample was treated with 120 µL of cell lysis buffer containing 1mM EDTA, 180 mM NaCl and RNAse A (Invitrogen, USA) at a concentration of 1.35Kunitz units/mL, for 2 hours at room temperature. CyQUANT^{®} GR dye was subsequently added to the RNAse-digested samples and the resulting fluorescence signals (excitation 480 nm, emission 520 nm) were measured to determine DNA concentrations with reference to a standard DNA calibration curve using a microplate reader (SPECTRAMAX^{®} M5, Molecular Devices, USA).

### 1.9. Encapsulation of microtissues in alginate hydrogel

As illustrated in **Fig. 1B****,** alginate microcapsules containing microtissues were produced using an electrostatic droplet generator following published protocols [Dang, T.T. et al., Biomaterials 34 (23): 5792-5801 (2013); Veiseh, O. et al., Nat Mater 14 (6): 643-651 (2015)]. Sodium alginate (PRONOVA UP LVG, FMC Biopolymer, Norway) was dissolved at 1.5% (w/v) in a 0.9 wt% NaCl solution. Microtissues were homogeneously suspended in 500 µL of the sodium alginate solution at a density of approximately 340-500 microtissues/mL and loaded into a 1 mL syringe. The microtissue-containing alginate suspension was extruded through a 20G blunt needle (SAI Infusion Technologies, USA) of the droplet generator into a bath of 20 mM BaCl₂ solution at a flow rate of 0.2 mL/min and a voltage of in a range of 5.0-6.0 kV. Alginate microcapsules were left to crosslink for 2 min before they were rinsed three times with glucose-free KRBH buffer. The diameters of the alginate hydrogel microcapsules produced were in the range of 800-1000 µm. Images of all encapsulated microtissues were independently examined by three researchers to determine the average number of compromised microtissues for quantification of the fraction of microtissue breakage post-encapsulation. Ten encapsulated intact microtissues and ten bare microtissue of each geometry were individually cultured in RPMI complete medium in separate wells in a 96-well plate. The wells containing the bare microtissues were previously coated with 2.3 wt% agarose which solidified to form a hydrogel layer at the bottom of the well before microtissues were added. Optical images of these individual microtissues were acquired at various time points to monitor and compare any change in their structural appearance.

### 1.10. Statistical analysis

All values are expressed as the mean ± standard error of the mean. The statistical significance of percentage of dead cells, metabolic activity per microtissues, volume and SA/Vol ratio of microtissues were determined using one-way ANOVA analysis with Tukey's post-hoc test software. Secreted insulin levels after exposure to 2.8 mM and 16.8 mM glucose solutions were compared using one-way repeated measures ANOVA analysis with Tukey's post-hoc test. P-values less than 0.05 were considered significant.

### EXAMPLE 2

### Formation of islet-like toroidal microtissues on hydrogel micromold

Microtissues with controlled geometries were fabricated using micromolded nonadhesive agarose hydrogel as shown in the schematic diagram of Fig. 1A. Agarose hydrogels have been used to produce three-dimensional (3D) multi-cellular aggregates due to its cytocompatibility, nonadherent property and ease of physical crosslinking at room temperature [Napolitano, A.P. et al., Biotechniques 43 (4): 494-500 (2007)]. In this example, agarose solution (2.3% w/w) was brought into contact with autoclaved silicone templates containing arrays of features with desired geometries and were left to solidify at room temperature to form micromolded agarose hydrogel. Upon removal, the agarose hydrogels contained precise negative replicates of the geometrical patterns on their corresponding silicone templates. Previous studies reported that dispersed rat hepatoma, human fibroblasts and human umbilical vein endothelial cells were able to aggregate into multi-cellular microtissues on micromolded nonadherent agarose hydrogels [Dean, D.M. et al., FASEB J 21 (14): 4005-4012 (2007); Napolitano, A.P. et al., Tissue Eng 13 (8): 2087-2094 (2007)]. In this study, rat insulinoma INS-1E cell line, due to its ability to secrete insulin in response to varying physiological glucose concentrations [Merglen, A. et al., Endocrinology 145 (2): 667-678 (2004)], was chosen to model β-cells which constitute the major cell type in native pancreatic islets. Suspension of monodispersed INS-1E cells was seeded into the recesses of micromolded agarose hydrogels where these cells sank due to gravity. The nonadhesive surface of agarose hydrogels prevented seeded cells from attaching to the bottom of the recesses, facilitating interaction between mono-dispersed cells which aggregates in the recess of the agarose mold to form 3D structures of desired geometry [Berridge, M. Tan, A. Protoplasma, 205: 74-82 (1998); Saelens, X. et al., Oncogene, 23: 2861-2874 (2004)]. This self-assembly of INS-1E cells was possibly regulated by extracellular calcium ions and E-cadherin, both of which had been reported to play an essential role in the modulation of cell-cell interaction to maintain the architecture of primary islets [Dahl, U. et al., Development 122 (9): 2895-2902 (1996); Rouiller, D.G. et al., Dev Biol 148 (1): 233-242 (1991)]. During the process of cellular aggregation, microtissues underwent contraction to form compact structures which were gently dislodged from the recesses of the agarose mold by pipetting and subsequently transferred into fresh culture medium.

### EXAMPLE 3

### Effect of initial cell seeding density on microtissue size and viability

The inventors evaluated the feasibility of fabricating INS-1E islet-like microtissues with different geometries and the ability to tune microtissue size by varying initial cell seeding density. The optical images in **Fig. 2A****(i-iv), B(i-iv)** and **C(i-iv)** showed the formation of microtissues with toroid, rod and spheroid geometries in the micromolded agarose hydrogels as dictated by the shapes of the recesses. **Fig. 2A****(v-viii), B(v-viii), C(v-viii)** are bright-field images of these microtissues after their retrieval from the agarose hydrogels by pipetting. The similar morphologies of the microtissues before and after retrieval confirmed that the structural integrity of most microtissues was not compromised by this process.

It was previously reported that cell viability decreased with increasing size of human islets [Komatsu, H. et al., PloS one 12 (8): e0183780 (2017)]. To quantitatively explore the relationship between the initial seeding density and the microtissue size, cells were seeded into the agarose hydrogels at various seeding densities between 3 to 10 × 10⁶ cells/ml. Immediately after retrieval from micromolded agarose hydrogel, microtissues were enzymatically dispersed into single cell suspension by incubation with trypsin, followed by gentle pipetting and staining with trypan blue. microtissues were enzymatically dispersed into single cells and characterized by trypan blue exclusion assay to quantify the percentage of dead cells. An automated cell was subsequently used to determine the number of cells in each microtissue. The percentage of dead cells was also calculated as the ratio of the number of dead cells to the total number of cells in the suspension. For each geometry, the cell number per microtissue increased with the initial cell seeding densities **(****Fig. 2D****, E, F**), demonstrating our ability to control microtissue size solely by changing initial seeding density. In addition, the percentage of dead cells in the microtissues increased with the cell number per microtissue (**Fig. 2D****, E, F**). This quantitative result was consistent with the optical images visually demonstrating that, for each geometry, the dark region comprising dead cells at the centre of microtissues also increased with initial cell seeding density (**Fig. 2A****, B, C**). This finding was in agreement with published studies reporting that cellular viability decreased with increasing size of human islets [Livoti, C.M. Morgan, J.R. Tissue Eng Part A, 16: 2051-2061 (2010)] and hepatocyte spheroids [Henquin, J.C. Diabetes, 49: 1751-1760 (2000)]. Both a computational stimulation model [Komatsu, H. et al., PloS one 12 (8): e0183780 (2017)] and a recent *in vitro* study [Sigmundsson, K. et al., Matrix Biol 70: 5-19 (2018)] suggested that hypoxia developed in human and mouse islets is the major factor implicated in the formation of dead cells at the core of these islets due to the low oxygen tension. Furthermore, in our experiments, for a similar percentage of cell death of about 13%, the number of cells per microtissue was approximately 14000, 7500 and 5500 for the toroid, rod and spheroid microtissues, respectively (**Fig. 2G**). This result demonstrated that, to achieve similar cell viability, toroid geometry supports cell packing into individual microtissues more efficiently than rod and spheroid geometries.

### EXAMPLE 4

### Effects of microtissue geometry on cell viability

The effect of microtissues geometry on cell viability was further evaluated by characterizing the metabolic activity of microtissues with toroid, rod and spheroid shapes. The cell number per microtissue was first standardized while varying microtissue geometry. The relationship between initial cell seeding density and cell number per microtissue for each geometry (**Fig. 2** **and** **Fig. 8**) was utilized to estimate the initial cell seeding densities for this volume standardization. We fabricated toroid, rod and spheroid microtissues with a similar number of cells per microtissue using initial cell seeding densities of 3.5, 5.8 and 9.3 million cells/ml, respectively. Microtissues were dispersed into single cell suspension after trypsinization, stained with trypan blue and counted by automated cell counter. The result in **Fig. 3A** confirmed that the average number of cells per microtissue for all 3 geometries was approximately 12000. The average percentage of dead cells was the lowest (11%) for toroid microtissues and the highest (29%) for spheroid microtissues (**Fig. 3B**). This was in agreement with the optical images (**Fig. 3C-E**) showing that the dark region at the core of the microtissues was smallest for toroid and largest for spheroid geometry. In addition to characterizing the relative percentage of live and dead cells, monitoring the metabolic health of live cells is also important for understanding the effects of microtissue geometry. In parallel experiments, WST-1 reagent was used to characterize the metabolic activity of live cells of intact microtissues. The tetrazolium salt WST-1 can be cleaved primarily at the external surface of live cells to form a water soluble formazan, whose concentration can be determined colorimetrically [Berridge M. and Tan, A. Protoplasma 205 (1-4): 74-82 (1998)]. **Fig. 3F** showed that toroid microtissues had much higher metabolic activity than rod and spheroid microtissues. **Fig. 3F** showed that toroid microtissues had a much higher metabolic activity than that of rod or spheroid microtissues. Interestingly, though the percentage of dead cells for spheroid microtissues was higher than that of rod microtissues **(****Fig. 3B****),** the metabolic activity of rod microtissues was the same as that of spheroid microtissues (**Fig. 3F****)**. Overall, these results **(****Fig. 3B-F**) demonstrated that toroidal shape was a better geometry to maintain the viability and metabolic activity of islet-like microtissues.

To explore the relationship between microtissue geometry and SA/Vol, toroid, rod and spheroid microtissues were approximated to ideal geometries which are torus, cylinder and sphere respectively. The volume of the microtissues were estimated using mathematical formulae for these ideal shapes and the dimensions of microtissues measured from optical images (**Fig. 9**). **Fig. 3G** suggested that, for the same standardized volume, toroid and rod microtissues had significantly higher SA/Vol compared to that of spheroid microtissues. This was consistent with the result that that the percentage of dead cells of both toroid and rod microtissues were lower than that of spheroid microtissues (**Fig. 3B**).

To further visualize the viability of microtissues, the inventors performed a fluorescent staining assay followed by confocal microscopy to visualize the 3D distribution of live and dead cells in the microtissues. In this assay, viable cells were stained by non-fluorescent cell-permeable Calcein-AM which is cleaved by intracellular esterases to produce green fluorescence. Concurrently, dead cells were stained by ethidium homodimer-1 which permeates compromised cell membrane to bind to DNA and produce red fluoresence. **Fig. 4A-C** are the bright-field images of the stained microtissues while **Fig. 4D-L** shows the combined projection of multiple images captured at different depths of the same microtissues. The green channel images (**Fig. 4D****, E, F**) confirmed that most cells on the surface of all three types of microtissues were alive. However, the red channel images (**Fig. 4G****, H, I**) revealed interesting differences in the distribution of dead cells in the interior of the microtissues of three geometries. Toroid microtissues have only a few dead cells which spread sporadically throughout the microtissues. On the contrary, more dead cells were detected in the interior of the rod microtissues **(****Fig. 4H**) at the same position as the dark cores in the bright field images (**Fig. 4B**) of the same microtissues as pointed at by the white arrows. Furthermore, the bright-field images (**Fig. 4A****, B**) also showed that toroid and rod microtissues retained their original appearance and structural integrity after several staining and rinsing steps during the live/dead assay. In contrast, a light grey circular area, as pointed at by the white arrows in **Fig. 4C****,** was observed at the center of each of several stained spheroid microtissues instead of a dark central core comprising of dead cells for unstained spheroids (**Fig. 3E**). This feature suggested that the dead cells at the core of these spheroid microtissues had been dislodged during the staining and rinsing process, allowing more light to penetrate through the core of the microtissues. The cellular debris from the core was probably discharged through small holes found on the surface of the spheroid microtissues (**Fig. 9**). A small fraction of dead cells remained adjacent to the outer live cell region, forming the circular black rim surrounding the central light gray region (**Fig. 4C**). This loss of dead cells from the core of some spheroid microtissues also accounts for a decrease in red fluorescence signal at the center of these microtissues (**Fig. 4I**), resulting in a red fluorescence signal comparable to that of rod microtissues, which did not suffer from the loss of dead cells during the staining and rinsing steps. The loss of dead cells from the core did not occur for unstained spheroid microtissues (**Fig. 3E**) which were not subjected to any fluorescent dye or rinsing process prior to the quantification of percentage of dead cells by trypsinzation and cell counting **(****Fig.3B****).** Hence, the dead cells at the core were still accounted for in the higher percentage of dead cells for spheroid microtissues compared to that of rod microtissues as reported in **Fig. 3B****.** Overall, the weaker mechanical properties of spheroid microtissues compared to that of toroid and rod structures correlated with the higher percentage of dead cells in these spheroids.

### EXAMPLE 5

### Effect of microtissue geometry on insulin secretion

Physiologically appropriate insulin secretion from therapeutic cell transplants is important for maintaining glucose homostasis since excessive secretion led to hypoglycemia and insufficient secretion gave rise to hyperglycemia [Henquin, J.C. Diabetes 49 (11): 1751-1760 (2000)]. The inventors performed a glucose-stimulated insulin secretion (GSIS) assay to investigate the effect of microtissue geometry on insulin secretion from the INS-1E microtissues. Microtissues were subjected to three consecutive incubations at glucose levels of 2.8 mM, 16.8 mM and 2.8mM to mimic alternate exposures to physiologically basal condition, hyperglycemic condition and a return to basal condition in diabetic patients respectively. The result showed that for each geometry, a greater amount of insulin was secreted in response to the higher glucose level during the second incubation compared to insulin secretion during the first exposure to lower glucose level (**Fig. 5**). The third incubation at the lower glucose level demonstrated a return to basal insulin level after the previous stimulation at the higher glucose level, confirming that the increased insulin secretion during the second incubation at high glucose level was not due to insulin dumping from distressed beta cells. Changing from a low to high glucose concentration led to an increase in insulin secretion within the range of 3.1 to 7.7-fold, comparable to results from a previous study reporting a 6.6-fold glucose stimulation index for INS-1E spheroid clusters [Merglen, A. et al., Endocrinology 145 (2): 667-678 (2004)].

### EXAMPLE 6

### Enhanced structural integrity of toroid microtissues encapsulated in alginate microcapsules

The inventors also investigated the feasibility of forming defect-free alginate microcapsules encapsulating islet-like microtissues and evaluated whether the structural integrity of these microtissues would be compromised by this encapsulation process. In this experiment, a mixture of alginate and microtissues was extruded through a 20G needle of an electrostatic droplet generator into a solution of Ba²⁺ ions where the microtissue-containing alginate droplets were crosslinked to form hydrogel microcapsules. **Fig. 6A-F** showed that microtissues of all three geometries could be encapsulated in uniform spherical alginate microcapsules, which were optically transparent and without significant surface deformation or visible irregularities as indicated by the blue arrows. Furthermore, despite their non-spheroidal geometry, toroid (**Fig. 6A** and **D**) and rod (**Fig. 6B** and **E**) microtissues did not result in alginate microcapsules with undesirable irregularities such as tails or deformed hydrogel surface, which might otherwise lead to increased recruitment of immune cells and subsequent fibrotic host response following in vivo transplantation [de Groot, M. et al., J Surg Res 121 (1): 141-150 (2004)]. For all three geometries, encapsulated microtissues were covered by the alginate hydrogel membranes without any visible irregular tissue protrusion **(****Fig. 6A-F),** providing adequate immuno-protection for the encapsulated microtissues *[*Ma, M. et al., Adv Healthc Mater 2 (5): 667-672 (2013*);* Chang, T.M. Nat Rev Drug Discov 4 (3): 221-235 (2005)]. Interestingly, a small fraction of encapsulated rod and spheroid microtissues was broken with a microtissue breakage ratio of ~5.5% and ~20.6% respectively (**Fig. 7**). In contrast, all toroid microtissues in the alginate microcapsules still retained their pre-encapsulation structural appearance with a breakage ratio of ~0% (**Fig. 7**)

The inventors also monitored the appearance of unencapsulated, bare toroid microtissues in comparison with encapsulated toroid microtissues which were cultured for 3 days following microtissue retrieval from micromolded agarose hydrogel and encapsulation in alginate microcapsules. Retrieved bare microtissues were cultured on top of a flat layer of solidified agarose coated on the bottom of a tissue culture plate to prevent their adherence to the treated polystyrene surface of the plate. **Fig. 8A** shows the optical images of a representative bare toroid microtissues and an encapsulated one at different time points while **Fig. 8B** summarizes the quantitative analysis of the corresponding changes in the inner diameter of these toroid microtissues. The lumen at the center **(Fig. 8A(i))** of bare toroid microtissue quickly closed after one day (**Fig. 8A(ii)** and **Fig. 8B**); and the initial toroid geometry of these microtissues changed into a spheroid shape which developed a center core of dead cells from day 2 onwards (**Fig. 8A(iii-iv)**). In contrast, toroid microtissues encapsulated in alginate hydrogel retained their center lumen till day 3 **(Fig. 8A(v-viii))** even though the lumen diameter decreased slightly **(****Fig. 8B****).** The data in **Fig. 8** demonstrated that encapsulation in crosslinked alginate hydrogel helped to preserve the toroid geometry of the microtissues.

Overall, this study has demonstrated that toroid microtissues fabricated from insulinoma INS-1E cell line exhibited enhanced viability, which should result in a lower number of microtissues required for glycemic correction in each recipient. This leads to an increase in the utilization efficiency of the transplanted islet tissue to potentially compensate for the loss of cell mass during islet dispersion. Furthermore, transplanting less microtissues also decreases the number of encapsulating hydrogel microcapsules and hence reduces the transplant volume. In addition, a lower percentage of dead cells in toroid microtissues also minimizes the formation of cellular debris which would otherwise elicit host immune response post-transplantation. Therefore, the advantage of dissociating islets into single cells and reassembling them into more viable microtissues potentially outweighs the disadvantage of islet mass loss during dispersion. A similar rationale has been adopted to support the dissociation of whole pancreas into individual islets to achieve preclinical success in blood glucose correction despite some islet mass loss [Ballinger W.F. and Lacy, P.E. Surgery 72 (2) (1972) 175-186].

Scaling up the fabrication process to mass produce toroid microtissues for clinical application may be achieved by cell passaging and seeding using an automated robotic liquid handling system. Recent studies have shown that interconnected incubators, liquid-handling system, centrifuges and microscopes via two central robotic arms were used for handling induced pluripotent stem cells [Paull, D. et al., Nat Methods 12 (9): 885-892 (2015); Conway, M.K. et al., J Vis Exp (99): e52755 (2015)]. Cell seeding and microtissue retrieval can be automated by similar robotic platforms with minimal manual intervention to enable high-throughput fabrication of toroid microtissues.

### EXAMPLE 7

### Fabrication of macrodevice with waffle-like structure containing microtissues

The planar hydrogel-based macrodevice of the disclosure has a waffle-like structure (**Fig. 12**) which consists of an array of microwells for entrapment of therapeutic microtissues and an interconnected network of sidewall containing vascular endothelial cells. Photolithographic microfabrication techniques were leveraged to design the network of sidewalls that guide the formation of an organized vascular network. Typically, the procedure shown in **Fig. 12** was used to fabricate the device. First, gelatin methacryloyl (GelMA) was patterned to form a waffle-inspired hydrogel network interspersing square or circular microwells of defined dimension. Briefly, a volume of 45 µL of GelMA prepolymer solution with I2959 photoinitiatior (0.5% w/v) was pipetted onto the surface of the lid of a Petri dish in between two spacers, each consisting of two stacked coverslips. To introduce vascular-inductive cells into the GelMA network of the macrodevice, human umbilical vascular endothelial cells (HUVECs) could be

mixed with the GelMA prepolymer before this mixture was dispensed on the petri dish. Afterwards, a glass slide (22 mm x 50 mm) was gently placed onto the GelMA solution in between the spacers. The 300 µm thickness of the spacer controls the height of the confined GelMA prepolymer film. Subsequently, a photomask with array of holes or transparent features with specified dimension and geometry was placed on the top of the glass slide. After 19 seconds of exposure to ultraviolet light (360-480 nm; 7.9 mW cm⁻²), the UV-exposed portion of the GelMA film was cross-linked to form the micropatterned network of sidewall. Afterwards, the glass slide bearing the UV-cross-linked GelMA pattern was rinsed with buffer solution to remove the uncross-linked GelMA residue that was not exposed to UV light, leaving behind microwells. By changing the design of the photomasks, various micropatterns with aligned arrays of either square or circular microwells could be fabricated as shown in **Fig. 13****.**

To encapsulate therapeutic microtissues into this device, a mixture of microtissues and alginate was dispensed onto the UV-cross-linked GelMA micropattern. For example, microtissues consisting of INS-1E cells were fabricated *ex situ* using micromolded non-adhesive agarose hydrogel (described in Example 2). After microtissues seeding, 20 mM BaCl₂ solution was added on top of the alginate-microtissue layer to crosslink the alginate for 10 minutes. Following alginate gelation, the whole macrodevice (1 cm × 1 cm) was gently detached from the glass slide with a surgical blade.

Alternatively, to induce *in situ* aggregation of monodispersed cells into microtissues (**Fig. 14**), 80 µL of monodispersed INS-1E cells suspension with a desired density were dispensed onto the UV-cross-linked GelMA micropattern. The seeded cells, which were evenly distributed in microwells of the GelMA micropattern, were incubated for 4 hours in 37°C and 5% CO₂ atmosphere. Next, excess media was added and the sample was incubated for another 20 hours until the cells aggregated into spherical or toroid microtissues depending on the chosen GelMA micropattern. For example, by designing a peg-like structure in each microwell (**Fig. 14**), a toroidal microtissue can be assembled in each microwell of the final device. Subsequently, the culture medium was removed and alginate solution was added on the microtissue-containing GelMA micropattern and cross-linked with barium chloride solution to form an immuno-isolating hydrogel layer.

### EXAMPLE 8

### Efficient and homogenous spatial distribution of microtissues by waffle-like macrodevice

Six macrodevices with different designs of GelMA network were evaluated for their performance in efficiently trapping and homogenously distributing microtissues (**Fig. 15A**). After seeding and encapsulating the microtissues, all macrodevices maintained their structural integrity (**Fig. 15A**). The number of microtissues encapsulated into each device was manually counted from optical images acquired post-encapsulation and was determined to be consistent at about approximately 730 per device (**Fig. 15B**).Two parameters were quantified and compared to select the device design with optimal performance. First, microtissue dwelling ratio, defined as the ratio of microtissues entrapped in the microwells to the total number of microtissues encapsulated in the entire device. A higher dwelling ratio indicates more efficient entrapment of microtissues inside the microwells as less microtissues stay on the GelMA sidewall. From **Fig. 15C****,** S-400, S-300 and C-400 macrodevices had significantly higher microtissue dwelling ratios of 91%, 89% and 76% respectively compared to other device designs.

Second, microtissue distribution ratio, defined as the ratio of the combined number of microtissues in all microwells of type M**n**, where **n** is the number of microtissue(s) per microwell, to the total number of microtissues encapsulated in the entire device. Entrapping only one microtissue per microwell is optimal to avoid aggregation of multiple microtissues and allowing perfusing of invidual microtissues by the surrounding vascularized network. Therefore, a higher distribution ratio for M1 microwells indicates a more homogenous microtissue distribution. Among the three device designs with the highest dwelling ratios, S-300 macrodevice had a significantly higher microtissue distribution ratio for M1 microwells as shown in **Fig. 15D****,** indicating that S-300 design achieved the most homogenous distribution of microtissues.

The optimal device S-300 was easily detached from the glass slide without structural compromise as demonstrated in **Fig. 16A****.** This S-300 device achieved a more homogenous spatial distribution of microtissues with much less clumping compared to the random aggregation of microtissues in a control device without any micropatterned design (**Fig. 16B**).

### EXAMPLE 9

### Viability and functional performance of encapsulated therapeutic microtissues in waffle-like macrodevice

The viability of encapsulated microtissues in the waffle-like macrodevice was assessed by fluorescent live/dead staining. **Fig. 17A** showed that most microtissues were stained green (light grey cells in lower right panels) demonstrating their viability post-encapsulation in our S-300 device. Both horizontal and vertical projection of the confocal image stacks verified the uniform arrangement of microtissues in regular arrays with equal spacing between adjacent microtissues. Subsequently, static glucose-stimulated insulin secretion test was performed by three consecutive incubations of the microtissue-containing macrodevice in KRBH containing 2.8 mM and then 16.8 mM glucose followed by 2.8 mM glucose at 37 °C for 2 hour per incubation and the amount of insulin secreted was quantified with insulin ELISA assay to evaluate their glucoseresponsivity. The encapsulated microtissue in the S-300 device demonstrated a 5-fold increase in insulin secretion after stimulation with high glucose concentration in the second incubation and the ability return to basal insulin secretion during the third incubation at low glucose concentration (**Fig. 17B**). This insulin secretion data confirmed that our design of waffle-inspired macrodevice could support the key therapeutic function of the encapsulated microtissues.

### EXAMPLE 10

### Formation of intra-device vasculature with spatially controlled pattern in waffle-like macrodevice

Our S-300 device was able to support formation of spatially controlled vascular network. Human vascular endothelial cells (HUVECs) incorporated in the micropatterned GelMA network demonstrated good viability after 7 days of culture (**Fig. 18A**), confirming that the use of photoinitiator and short UV exposure time did not significantly affect cell viability. HUVECs was also examined by immunofluorecent staining of CD31, which is a platelet endothelial cell adhesion molecule 1 (PECAM-1) constituting endothelial cell intercellular junctions and is often localized to the lumen-facing surface blood vessels [Albelda, SM. et al., The Journal of cell biology.;114(5): 1059-1068 (1991); DeLisser, HM. et al., Am J Pathol. 151(3): 671-677 (1997)]. Positive staining of CD31 (**Fig. 18B**) demonstrated the vascular-inductive potential of the incorporated HUVECs in our S-300 device.

### EXAMPLE 11

### Viability and function of co-loaded therapeutic microtissues and vascular-inductive cells in waffle-like macrodevice

When both vascular-inductive HUVECs and therapeutic insulin-secreting microtissues were incorporated in the S-300 device, both cell types maintained desirable viability as verified by live/dead fluorescence assay (**Fig. 18C**) after the microtissue-loaded device was placed in culture medium overnight. The result from GSIS assay in **Fig. 18D** demonstrated 6-fold increase in insulin secretion when the microtissue-loaded device was exposed to 16.8 mM glucose solution compared to the secretion level at 2.8 mM glucose solution. The insulin secretion form encapsulated microtissue was also able to return to basal level during the third incubation after glucose stimulation. The observed glucose stimulation index was characteristic of unencapsulated INS-1E spheroids and was similar to the index for the device without HUVECs in Example 9, confirming that the encapsulated microtissues were able to maintain their therapeutic function when concurrently with vascular-inductive cells in the waffle-like macrodevice.

### EXAMPLE 12

### Inducing assembly of monodispersed cells for in situ formation of spatially distributed microtissues in waffle-like macrodevice

While the S-300 macrodevice was able to achieve a high microtissue dwelling ratio of about 89%, a fraction of about 11% of all microtissues dispensed onto the device during fabrication ended up on the GelMA sidewall. To maximize the dwelling ratio and ensuring that each microwell contain one microtissue, the inventors adopted an alternative approach to induce assembly of monodispersed cells for *in situ* formation of microtissues in individual microwells. Suspension of monodispersed cells, instead of microtissues, was dispensed onto the micropatterned GelMA network and the individual cells easily fell into the depression of the microwells or migrated into the microwells. After about 24 hours of self-assembly, a multicellular cluster were formed in each microwell with no cluster falling outside of the microwell onto the GelMA sidewall (**Fig. 19**). With this approach, the ultimate microtissue dwelling ratio and the microtissue distribution ratio for M1 microwell both reached their theoretical limits of almost 100%. When a micropattern was re-designed to include a peg in the center of each microwell, the monodispersed cells self-assemble around the peg to form a toroid microtissue in each well as shown in **Fig. 20**. These results demonstrate the versatility of the waffle-inspired macrodevice design to facilitate *in situ* assembly of monodispersed cells to form microtissues with desired geometry.

An advantage of our device compared to existing methods is the ability to improve spatial distribution of the encapsulated microtissues while providing

endothelial cells for facilitating vascularization in an interconnected network which perfuses individual microtissues. Homogenous distribution of the microtissues encapsulated in our device prevents their clumping to potentially facilitate nutrients and oxygen transportation and enhance cell survival [Bochenek, MA. et al., Nature Biomedical Engineering. 2(11): 810 (2018); O'sullivan, ES. et al., Endocrine reviews. 32(6): 827-844 (2011)]. Encapsulating the therapeutic microtissues in alginate hydrogel also reduces the risk of excessive protrusion, which might otherwise elicit severe inflammatory responses followed by fibrosis of the implanted device and subsequent tissue death [De Vos, P. et al., Transplantation. 62(7): 893-899 (1996); Bhujbal, SV. et al., Scientific reports. 4: 6856 (2014)].

### EXAMPLE 13

### Optimal design parameters of waffle-like macrodevice to facilitate in situ formation of toroid microtissues

To facilitate microtissue formation in waffle-inspired macrodevice, which comprises of multiple square microwells each with a cylindrical peg at its center, key design parameters include microwell width dimension and peg diameter (**Fig. 21**). Various combinations of peg diameters between 50 µm and 150 µm and microwell side width dimensions between 400 µm and 600 µm were investigated.

The inventors observed that microtissues can be formed with the most uniform, complete toroid geometry and good viability in the macrodevice with wells having a width dimension or diameter of 600 µm and peg width or diameter of 150 µm (**Fig. 22**). In contrast, small peg diameters of 50 µm caused seeded cells to settle on the upper surface of the peg and resulted in formation of disc-shaped microtissues without the central lumens instead of the desired toroid shape.

### EXAMPLE 14

### Optimal initial cell loading to maintain viability of toroid microtissues in waffle-like macrodevice

Various amounts of cells were seeded into the same micropatterned GelMA hydrogel network (1cm x 1cm) comprising of multiple microwells each with a side- width dimension of 500 µm and peg diameter of 150 µm. Initial cell loading or initial numbers of live cells seeded into each micropatterned GelMA hydrogel ranged from 0.3 to 1.3 million cells per cm² were investigated (**Fig. 23**). Twenty-four hours after cell seeding, the microtissue-containing GelMA hydrogel was gently detached from the glass slide with a surgical blade after removal of culture media. The detached GelMA hydrogel and associated microtissues were trypsinized to release monodispersed cells for quantification and viability assessment of the amount of retrieved cells by trypan blue staining.

The inventors found that cell viability can be maintained at more than 90% for all initial cell loading numbers (**Fig. 24A**). Cell usage yield or efficiency, calculated as the ratio of retrieved live cells to the initial cell seeding number, was optimal at an initial seeding density of 0.3 to 0.7 million (**Fig. 24B**) due to minimal formation of cell sheet at the edge of the device. Excessive cell loading caused loss of cells from the device due to the formation of cell sheets on the edges of macrodevices when 0.9 to 1.3 million cells were initially seeded per cm² (**Fig. 24A**)

### EXAMPLE 15

### Static glucose-stimulated insulin secretion assay from waffle-like macrodevice encapsulating toroid microtissues

A glucose-stimulated insulin secretion (GSIS) assay was performed to evaluate the function of the toroid microtissues assembled *in situ* and encapsulated in waffle-like macrodevice (as described in Example 7). After the removal of all culture medium, waffle-like macrodevice containing toroid microtissues was rinsed with glucose-free medium twice to remove residual insulin. The device was preincubated with Krebs Ringer buffer HEPES (KRBH) (135 mM NaCl, 5 mM NaHCO₃, 0.5 mM NaH₂PO₄, 3.6 mM KCI, 1.5 mM CaCl₂, 0.5 mM MgCl₂, and 10 mM HEPES, pH 7.4. BSA (0.1%)) containing 2.8 mM glucose for 2 hours at 37 °C. Afterwards, the *in vitro* insulin secretion was evaluated by consecutive incubations of the microtissue-containing macrodevice in containing 2.8 mM, 16.8 mM and 2.8 mM glucose at 37 °C for 2 hours to mimic alternate exposures to physiologically basal condition, hyperglycemic condition and a return to basal condition in diabetic patients respectively. After each incubation, the residual insulin was removed by rinsing samples twice with glucose-free KRBH buffer between consecutive incubations. At the end of each incubation, 100 µL of the supernatant was removed and stored at -20 °C for subsequent analysis. The insulin concentrations was measured by Ultrasensive Insulin ELISA (ALPCO Diagnostics, USA).

The result showed that a higher average amount of insulin was secreted during the second incubation at high glucose concentration compared to insulin secretion during the first and third incubations at lower glucose concentration (**Fig. 25**). This result illustrated that toroid microtissues assembled and encapsulated in the macrodevice retained the ability to respond to glucose concentration *in vitro,* demonstrating a 4.3-fold increase in insulin secretion after glucose stimulation.

### EXAMPLE 16

### Mechanical and structural stability achieved by interlocking modular components of waffle-like macrodevice

The macrodevice can be fabricated with strong mechanical property to sustain handling with tweezers. Minimal deformation of the waffle arrangement is observed (**Fig. 26A-B**). As illustrated in (**Fig. 27**), the "lock" component consisted of an interconnected micropatterned GelMA hydrogel network, which served as dividing sidewalls separating evenly spaced microwells. The "key" component consisted of therapeutic microtissues encapsulated in an immuno-isolatory alginate hydrogel that fitted into the microwells of the lock component. The interlocking design of our waffle-inspired device minimized the risk of the two components detaching (**Fig. 31B**) to ensure the proximity of the vascular-inductive cells in the lock component and the encapsulated therapeutic microtissues in the key component.

### EXAMPLE 17

### Mechanical and structural stability of toroid-loaded macrodevice after subcutaneous transplantation into diabetic mice

As per known methods [Dang, T.T et al., Biomaterials 34 (23): 5792-5801(2013)], diabetic C57BL/6J mice were generated from healthy mice by multiple low-dose daily injections of Streptozotocin (STZ) to induce damage to their pancreatic beta cells (**Fig. 28**). The waffle-like macrodevice containing microtissues assembled *in situ* from insulinoma (INS1E) cells was subcutaneously transplanted into these diabetic mice to evaluate whether the cells embedded in the macrodevice can secrete insulin to correct blood glucose level. It was observed that the macrodevice can be transplanted into the mice and retrieved after 9 days without compromising its mechanical properties or fusion of microtissues (**Fig. 29**).

### EXAMPLE 18

### Effect of toroid microtissues assembled and encapsulated in waffle-like macrodevice on blood glucose level in diabetic mice

Using the method in Example 17, the inventors have established a chemically induced diabetic mouse model to evaluate the therapeutic function of INS-1E toroid microtissues assembled and encapsulated in waffle-like macrodevices. These microtissue-containing waffle-like macrodevices, which were fabricated as described in Example 7, were subcutaneously transplanted onto the dorsal side of diabetic C57/B6 mice to establish a marginal/minimal cell mass model, which corresponds to the minimum amount of cells required to correct blood sugar level in 50% of the mice.

The results show that an initial cell seeding number of just 1 to 2 million of INS-1E cells per waffle-like macrodevice was sufficient to lower the blood glucose level of diabetic mice after 1 day (**Fig. 30**). This was superior compared to the method of Coronel, MM. et al., Biomaterials 210: 1-11 (2019), which required 2.5 million MIN6 cells per mouse to lower blood glucose below 300 mg/dL after 17 days post-transplantation using a disk-shaped macrodevice.

### EXAMPLE 19

### Effect of toroid microtissues encapsulated in alginate microcapsules on blood glucose level in diabetic mice

Marginal cell mass (about 2.4 million INS-1E cells) of 200 toroid microtissues, which were fabricated and encapsulated in alginate microcapsules as described in Example 1, were transplanted to the intraperitoneal space of each diabetic mouse established as described in Example 17. Results show that the toroid microtissues can lower blood glucose level of diabetic mice to below 300 mg/dL just 1day post-transplantation (**Fig. 31**).

### EXAMPLE 20

### Fabrication of waffle-like macrodevice encapsulating primary rat islet microtissues

Primary rat islets were isolated from Sprague-Dawley rats by collagenase digestion and purification with Ficoll gradient as per known methods [O'Sullivan, E. et al., Diabetologia 53 (5): 937-945] (2010)]. These primary rat islets were mixed with alginate and the alginate-islet mixture was dispensed on micropatterned GelMA hydrogel network. The array of microwells of the GelMA hydrogel network facilitated homogenous distribution of the therapeutic microtissues by entrapping each islet per microwell for the majority of the islets (**Fig. 32A**). The islet-containing waffle-like macrodevice was further coated with an external alginate hydrogel layer followed by crosslinking in barium chloride solution. The macrodevice maintained the homogenous distribution of islets after its detachment from the glass slide by a surgical blade (**Fig. 32B**).

### Summary

We demonstrated that the geometry of microtissues plays a significant role in microtissue viability. Compared with rod and spheroid microtissue, toroid microtissues exhibited enhanced cell viability and metabolic activity. In addition, this microtissue geometry did not affect the insulin secretion of INS-1E cell lines. Furthermore, toroid microtissues retained their intact structure following encapsulation in immuno-isolatory hydrogel and reduced blood glucose levels when implanted intraperitoneally in STZ-induced diabetic mice.

In addition, the disclosed waffle-shaped hybrid hydrogel macrodevice has several advantages, including (1) improving spatial distribution of microtissues, (2) supporting vascularization of human-derived endothelial cells encapsulated in waffle-inspired interconnected network, (3) consisting of interlocking components to enable stability and proximity of the vascular-inducing network and the immuno-isolatory hydrogel component with therapeutic microtissues and (4) inducing *in situ* aggregation of monodispersed cells into homogenously distributed microtissues of desired geometry. We showed that the planar biocompatible hydrogel-based macrodevice comprising an array of microwells, each containing single islet toroid, and an interconnected network of sidewalls containing vascular endothelial cells reduced blood glucose levels when implanted intraperitoneally in STZ-induced diabetic mice.

### References

Any listing or discussion of an apparently prior-published document in this specification should not necessarily be taken as an acknowledgement that such document is part of the state of the art or is common general knowledge.
Albelda SM, Muller WA, Buck CA, Newman PJ. Molecular and cellular properties of PECAM-1 (endoCAM/CD31): a novel vascular cell-cell adhesion molecule. The Journal of cell biology. Sep 1991;114(5): 1059-1068.
Avgoustiniatos ES, Colton CK. Effect of External Oxygen Mass Transfer Resistances on Viability of Immunoisolated Tissue a. Annals of the New York Academy of Sciences. 1997; 831(1 Bioartificial Organs: Science, Medicine, and Technology): 145-166.
Ballinger, WF. Lacy, PE. Transplantation of intact pancreatic islets in rats. Surgery 72 (2): (1972) 175-186.
Baranski JD, Chaturvedi RR, Stevens KR, et al. Geometric control of vascular networks to enhance engineered tissue integration and function. Proceedings of the National Academy of Sciences. 2013;110(19): 7586-7591.
Barkai, U. Weir, GC. et al., Enhanced oxygen supply improves islet viability in a new bioartificial pancreas. Cell Transplant 22 (8): (2013) 1463-1476.
Berridge, M. Tan, A. Trans-plasma membrane electron transport: a cellular assay for NADH-and NADPH-oxidase based on extracellular, superoxide-mediated reduction of the sulfonated tetrazolium salt WST-1. Protoplasma 205 (1-4): (1998) 74-82.
Bertuzzi, F. Marzorati, S. et al., Tissue factor and CCL2/monocyte chemoattractant protein-1 released by human islets affect islet engraftment in type 1 diabetic recipients. J Clin Endocrinol Metab 89 (11): (2004) 5724-5728.
Bhujbal SV, De Haan B, et al., A novel multilayer immunoisolating encapsulation system overcoming protrusion of cells. Scientific reports. 2014; 4: 6856.
Bochenek MA, Veiseh O, et al. Alginate encapsulation as long-term immune protection of allogeneic pancreatic islet cells transplanted into the omental bursa of macaques. Nature Biomedical Engineering. 2018; 2(11): 810.
Boettler T, Schneider D, Cheng Y, et al. Pancreatic Tissue Transplanted in TheraCyte Encapsulation Devices Is Protected and Prevents Hyperglycemia in a Mouse Model of Immune-Mediated Diabetes. Cell Transplant. 2016; 25(3): 609-614.
Bosco, D. Orci, L. Meda, P. Homologous but not heterologous contact increases the insulin secretion of individual pancreatic B-cells. Exp Cell Res 184 (1): (1989) 72-80.
Bruni A, Gala-Lopez B, et al., Islet cell transplantation for the treatment of type 1 diabetes: recent advances and future challenges. Diabetes, metabolic syndrome and obesity: targets and therapy. 2014; 7: 211.
Chang, TM. Therapeutic applications of polymeric artificial cells. Nat Rev Drug Discov 4 (3): (2005) 221-235.
Chen X, Aledia AS, Ghajar CM, et al. Prevascularization of a fibrin-based tissue construct accelerates the formation of functional anastomosis with host vasculature. Tissue Engineering Part A. 2008; 15(6): 1363-1371.
Colton CK. Oxygen supply to encapsulated therapeutic cells. Advanced drug delivery reviews. 2014; 67: 93-110.
Conway, MK. Gerger, MJ. Balay, EE. et al., Scalable 96-well plate based iPSC culture and production using a robotic liquid handling system. J Vis Exp (99): e52755 (2015).
Coronel, MM. et al., Oxygen generating biomaterial improves the function and efficacy of beta cells within a macroencapsulation device Biomaterials 210: (2019) 1-11.
Dahl, U. Sjodin, A. Semb, H. Cadherins regulate aggregation of pancreatic beta-cells in vivo. Development 122 (9): (1996) 2895-2902.
Dang, TT. Thai, AV. Cohen, J. et al., Enhanced function of immuno-isolated islets in diabetes therapy by co-encapsulation with an anti-inflammatory drug. Biomaterials 34 (23): (2013) 5792-5801.
Dean, D.M. Napolitano, A.P. et al., Rods, tori, and honeycombs: the directed self-assembly of microtissues with prescribed microscale geometries. FASEB J 21 (14): (2007) 4005-4012.
DeLisser HM, Christofidou-Solomidou M, Strieter RM, et al. Involvement of endothelial PECAM-1/CD31 in angiogenesis. Am J Pathol. Sep 1997;151(3): 671-677.
Glicklis, R. Merchuk, JC. Cohen, S. Modeling mass transfer in hepatocyte spheroids via cell viability, spheroid size, and hepatocellular functions. Biotechnol Bioeng 86 (6): (2004) 672-680.
de Groot, M. Schuurs, TA. van Schilfgaarde, R. Causes of limited survival of microencapsulated pancreatic islet grafts, J Surg Res 121 (1) (2004) 141-150.
Desai, T. Shea, L.D. Advances in islet encapsulation technologies. Nat Rev Drug Discov 16 (5): (2017) 338-350.
De Vos, P. De Haan, et al., Association between capsule diameter, adequacy of encapsulation, and survival of microencapsulated rat islet allografts1. Transplantation. 1996;62(7): 893-899.
Elliott, R.B. Escobar, L. et al., Live encapsulated porcine islets from a type 1 diabetic patient 9.5 yr after xenotransplantation, Xenotransplantation 14 (2): (2007) 157-161.
Enmon Jr, RM. O'Connor, KC. Et al., Dynamics of spheroid self-assembly in liquidoverlay culture of DU 145 human prostate cancer cells. Biotechnol Bioeng 72 (6): (2001) 579-591.
Gao, K. Kumar, P. Cortez-Toledo, E. et al. Potential long-term treatment of hemophilia A by neonatal co-transplantation of cord blood-derived endothelial colony-forming cells and placental mesenchymal stromal cells. Stem cell research & therapy. Jan 22 2019;10(1): 34.
Gionet-Gonzales, MA. Leach, JK. Engineering principles for guiding spheroid function in the regeneration of bone, cartilage, and skin. Biomedical materials. Mar 21 2018; 13(3): 034109.
Glicklis, R. Merchuk, JC. Cohen, S. Modeling mass transfer in hepatocyte spheroids via cell viability, spheroid size, and hepatocellular functions. Biotechnol Bioeng. Jun 20 2004;86(6): 672-680.
Grigoryan, B. et al., Multivascular networks and functional intravascular topologies within biocompatible hydrogels. Science 2019, 364: 458.
Henquin, J.C. Triggering and amplifying pathways of regulation of insulin secretion by glucose. Diabetes 49 (11): (2000) 1751-1760.
Hering, BJ. Clarke, WR. et al., Clinical Islet Transplantation, Phase 3 Trial of Transplantation of Human Islets in Type 1 Diabetes Complicated by Severe Hypoglycemia. Diabetes Care 39 (7): (2016) 1230-1240.
Hilderink, J. Spijker, S. et al., Controlled aggregation of primary human pancreatic islet cells leads to glucose-responsive pseudoislets comparable to native islets. J Cell Mol Med 19 (8): (2015) 1836-1846.
lonescu-Tirgoviste, C. Gagniuc, PA. et al., A 3D map of the islet routes throughout the healthy human pancreas, Sci Rep 5: (2015) 14634.
Jansson, L. Hellerstrom, C. Glucose-induced changes in pancreatic islet blood flow mediated by central nervous system. Am J Physiol 251 (6 Pt 1): (1986) E644-647.
Kilimnik, G. Jo, J. et al., Quantification of islet size and architecture. Islets 4 (2): (2012) 167-172.
Kim, A. Miller, K. et al., Islet architecture: A comparative study. Islets 1 (2): (2009) 129-136.
Köllmer, M. Appel, AA. et al., Long-term function of alginate-encapsulated islets. Tissue Eng Part B Rev 22 (1): (2015) 34-46.
Komatsu, H. Cook, C. et al., Oxygen environment and islet size are the primary limiting factors of isolated pancreatic islet survival. PloS one 12 (8): (2017) e0183780.
Lee BR, Hwang JW, Choi YY, et al., In situ formation and collagen-alginate composite encapsulation of pancreatic islet spheroids. Biomaterials. 2012;33(3): 837-845.
Levenberg S, Rouwkema J, Macdonald M, et al., Engineering vascularized skeletal muscle tissue. Nature biotechnology. 2005;23(7): 879.
Lim, F. Sun, A.M. Microencapsulated Islets as Bioartificial Endocrine Pancreas. Science 210 (4472): (1980) 908-910.
Livoti, CM. Morgan, JR. Self-assembly and tissue fusion of toroid-shaped minimal building units. Tissue Eng Part A 16 (6): (2010) 2051-2061.
Ma, M. Chiu, A. et al., Core-shell hydrogel microcapsules for improved islets encapsulation. Adv Healthc Mater 2 (5): (2013) 667-672.
Mendelsohn, A.D. Nyitray, C. Sena, M. Desai, T.A. Size-controlled insulin-secreting cell clusters. Acta Biomater 8 (12): (2012) 4278-4284.
Merglen, A. Theander, S. et al., Glucose sensitivity and metabolism-secretion coupling studied during two-year continuous culture in INS-1E insulinoma cells. Endocrinology 145 (2) (2004) 667-678.
Napolitano, AP. Dean, DM. et al., Scaffold-free three-dimensional cell culture utilizing micromolded nonadhesive hydrogels. Biotechniques 43 (4): (2007) 494-500.
Napolitano, AP. Chai, P. Dean, DM. Morgan, JR. Dynamics of the self-assembly of complex cellular aggregates on micromolded nonadhesive hydrogels. Tissue Eng 13 (8): (2007) 2087-2094.
O'Sullivan, E. Johnson, A. et al., Rat islet cell aggregates are superior to islets for transplantation in microcapsules. Diabetologia 53 (5): (2010) 937-945.
O'sullivan ES, Vegas A, Anderson DG, Weir GC. Islets transplanted in immunoisolation devices: a review of the progress and the challenges that remain. Endocrine reviews. 2011;32(6): 827-844.
Paull, D. Sevilla, A. et al., Automated, high-throughput derivation, characterization and differentiation of induced pluripotent stem cells. Nat Methods 12 (9): (2015) 885-892.
Pellegrini, S. Cantarelli, E. et al., The state of the art of islet transplantation and cell therapy in type 1 diabetes. Acta Diabetol 53 (5): (2016) 683-691.
Rago, AP. Chai, PR. Morgan, JR. Encapsulated arrays of self-assembled microtissues: an alternative to spherical microcapsules. Tissue Eng Part A 15 (2): (2008) 387-395.
Ramachandran, K. Williams, SJ. et al., Engineering islets for improved performance by optimized reaggregation in a micromold. Tissue Eng Part A 19 (5-6): (2012) 604-612.
Reynolds, TD. Mitchell, SA. Balwinski, KM. Investigation of the effect of tablet surface area/volume on drug release from hydroxypropylmethylcellulose controlled-release matrix tablets. Drug Dev Ind Pharm 28 (4): (2002) 457-466.
Rouiller, DG. Cirulli, V. Halban, PA. Uvomorulin mediates calcium-dependent aggregation of islet cells, whereas calcium-independent cell adhesion molecules distinguish between islet cell types. Dev Biol 148 (1): (1991) 233-242.
Rouwkema J, Khademhosseini A. Vascularization and Angiogenesis in Tissue Engineering: Beyond Creating Static Networks. Trends Biotechnol. Sep 2016;34(9): 733-745.
Saelens, X. Festjens, N. et al., Toxic proteins released from mitochondria in cell death. Oncogene 23 (16): (2004) 2861-2874.
Saisho, Y. Pancreas volume and fat deposition in diabetes and normal physiology: consideration of the interplay between endocrine and exocrine pancreas. Rev Diabet Stud 13 (2-3): (2016) 132.
Schell, JY. Wilks, BT. et al., Harnessing cellular-derived forces in self-assembled microtissues to control the synthesis and alignment of ECM. Biomaterials 77: (2016) 120-129.
Shapiro, AM. Pokrywczynska, M. Ricordi, C. Clinical pancreatic islet transplantation. Nat Rev Endocrinol 13 (5): (2017) 268-277.
Sigmundsson, K. Ojala, JRM. et al., Culturing functional pancreatic islets on alpha5-laminins and curative transplantation to diabetic mice. Matrix Biol 70: (2018) 5-19.
Song, S. Roy, S. Progress and challenges in macroencapsulation approaches for type 1 diabetes (T1D) treatment: Cells, biomaterials, and devices. Biotechnol Bioeng. Jul 2016;113(7): 1381-1402.
Tuch, BE. Keogh, GW. et al., Safety and viability of microencapsulated human islets transplanted into diabetic humans. Diabetes care 32 (10): (2009) 1887-1889.
Ungphaiboon, S. Attia, D. et al., Materials for microencapsulation: what toroidal particles ("doughnuts") can do better than spherical beads. Soft Matter 6 (17): (2010) 4070-4083.
Veiseh, O. Doloff, JC. et al., Size- and shape-dependent foreign body immune response to materials implanted in rodents and non-human primates. Nat Mater 14 (6): (2015) 643-651.
Wilson, JT. Chaikof, EL. Challenges and emerging technologies in the immunoisolation of cells and tissues. Adv Drug Del Rev 60 (2): (2008) 124-145.

## Claims

1. A planar biocompatible hydrogel-based macrodevice comprising an array of microwells and a therapeutic microtissue within each of a plurality of said microwells, wherein;
a)said macrodevice comprises a component comprising a "lock" component of an interconnected network of cross-linked hydrogel, which serves as dividing sidewalls separating evenly spaced microwells, wherein each microwell in the array comprises at least one side wall and is configured to encapsulate a single therapeutic microtissue; and
b) said macrodevice comprises a "key" component comprising therapeutic microtissues in an immuno-isolatory hydrogel that, when cross-linked, are entrapped in the microwells, and
wherein said immuno-isolatory hydrogel "lock" component of b) interlocks with the interconnected hydrogel network "key" component of a) through complementary topography features that fit together in a lock-and-key configuration and
wherein said planar biocompatible hydrogel-based macrodevice further comprises a coating of an immuno-isolatory hydrogel that encapsulates the macrodevice.

2. The planar biocompatible hydrogel-based macrodevice of claim 1, wherein the at least one side wall of each of a plurality of said microwells further comprise vascular endothelial cells.

3. The planar biocompatible hydrogel-based macrodevice of claim 1 or 2, wherein the coating of immuno-isolatory hydrogel is an alginate hydrogel.

4. The planar biocompatible hydrogel-based macrodevice of any one of claims 1 to 3, wherein each of said microwells further comprises a peg arranged to guide the formation of a toroid-shaped microtissue.

5. The planar biocompatible hydrogel-based macrodevice of any one of claims 1 to 3, wherein the therapeutic microtissue is toroid-shaped.

6. The planar biocompatible hydrogel-based macrodevice of any one of claims 1 to 4, wherein the microtissue comprises secretory, structural or metabolic cells.

7. The planar biocompatible hydrogel-based macrodevice of any one of claims 1 to 5, wherein the microtissues comprise cells selected from the group comprising islets of Langerhans cells, hepatocytes, bone marrow mononuclear cells, mesenchymal stem cells, mobilized peripheral blood mononuclear cells, endothelial progenitor cells, follicular cells, Leydig cells, ovarian cells, neural stem cells, human embryonic stem cells, pluripotent stem cells, induced pluripotent stem cells (iPSCs), skeletal myoblasts, cardiomyoblasts, and genetically engineered cells for growth hormone deficiency or haemophilia.

8. The planar biocompatible hydrogel-based macrodevice of claim 7, wherein the cross-linked hydrogel in a) is GelMA and/or the immuno-isolatory hydrogel in b) is alginate.

9. The planar biocompatible hydrogel-based macrodevice of any one of claims 1 to 8,
wherein each of said microwells has a width dimension in the range of 100 µm to 1000 µm, preferably 500 µm.

10. The planar biocompatible hydrogel-based macrodevice of any one of claims 4 to 9,
wherein the peg has a width dimension in the range of 50 µm to 150 µm, preferably the peg has a width dimension of 100 µm, and/or
wherein the peg has a width dimension of 100 µm and the width dimension of each of said microwells is 500 µm.

11. The planar biocompatible hydrogel-based macrodevice of any one of claims 1 to 10, wherein each of the said therapeutic microtissues:
a) was added to the said microwells, or
b) was generated within the said microwells from a cell suspension, wherein preferably the cell suspension comprises about 0.5 to 1.5 million cells per cm² of macrodevice.

12. A method of manufacturing a planar biocompatible hydrogel-based macrodevice of any one of claims 1 to 11, comprising the steps;
a) hydrogel prepolymer is dispensed onto a surface;
i) a photomask with an array of holes or transparent features of specified geometry and dimensions is placed over the dispensed mixture, and
ii) the mixture is exposed to UV light or solution containing cross-linker to crosslink an exposed portion of the hydrogel prepolymer, to form a micropatterned network of sidewalls, and
iii) the crosslinked hydrogel pattern is rinsed to remove non-crosslinked hydrogel residue, leaving behind microwells; or
b) hydrogel prepolymer is dispensed onto a surface using 3D printing and cross-linked to form a micropatterned network of sidewalls defining microwells;
c) adding a mixture of microtissues and cross-linkable immuno-isolating hydrogel onto the crosslinked hydrogel micropattern of a) or b), and crosslinking the immuno-isolating hydrogel; or
d) adding a suspension of cells to the microwells and culturing the cells under suitable conditions until the cells aggregate into desired microtissue shape depending on the chosen cross-linked hydrogel micropattern, and
(i) adding cross-linkable immuno-isolating hydrogel onto the cross-linked hydrogel micropattern, and
(ii) crosslinking the immuno-isolating hydrogel,
wherein the planar biocompatible hydrogel-based macrodevice comprises a single microtissue per microwell, and further comprising a step e) encapsulating the macrodevice and microtissues in a biocompatible hydrogel layer, such as an alginate hydrogel layer.

13. The method of claim 12, wherein the cells are seeded onto the macrodevice at about 0.3 to 1.5 million cells per cm² of macrodevice.

14. The method of any one of claims 12 or 13, wherein in step a) or b) a mixture of hydrogel prepolymer and vascular endothelial cells is dispensed onto said surface and wherein said vascular endothelial cells are embedded in the microwell side walls.

15. The method of any one of claims 12-14, wherein the hydrogel prepolymer in step a) or b) is GelMA and the cross-linkable immuno-isolating hydrogel in step c) or d)(i) is alginate.

16. A planar biocompatible hydrogel-based macrodevice of any one of claims 1 to 11 for the treatment of a disease in a subject, preferably wherein the macrodevice or the composition comprises microtissues that secrete insulin to treat diabetes, and/or wherein the macrodevice or the composition comprises microtissues that comprise islet cells.

17. A kit comprising a planar biocompatible hydrogel-based macrodevice of any one of claims 1 to 11.

## Patentansprüche

1. Planare, biologisch verträgliche Makrovorrichtung auf Hydrogel-Basis, die eine Anordnung von Mikrowells und ein therapeutisches Mikrogewebe innerhalb jedes der Vielzahl von Mikrowells umfasst, wobei:
a) die Makrovorrichtung eine Komponente umfasst, die eine "Schloss"-Komponente aus einem in sich zusammenhängenden Netzwerk aus vernetztem Hydrogel umfasst, die als trennende Seitenwände dient, die gleichmäßig beabstandete Mikrowells voneinander trennt, wobei jeder Mikrowell in der Anordnung zumindest eine Seitenwand umfasst und dazu ausgelegt ist, ein einzelnes therapeutisches Mikrogewebe einzukapseln, und
b) die Makrovorrichtung eine "Schlüssel"-Komponente umfasst, die therapeutische Mikrogewebe in einem immunisolierenden Hydrogel umfasst, das bei seiner Vernetzung in den Mikrowells eingeschlossen wird; und
wobei die "Schlüssel"-Komponente aus immunisolierendem Hydrogel aus b) und die "Schloss"-Komponente aus dem in sich zusammenhängenden Netzwerk aus Hydrogel aus a) durch komplementäre Topographie-Merkmale ineinandergreifen, die in einer Schloss-Schlüssel-Konfiguration zusammenpassen; und
wobei die planare, biologisch verträgliche Makrovorrichtung auf Hydrogel-Basis weiters eine Beschichtung aus einem immunisolierenden Hydrogel umfasst, das die Makrovorrichtung einkapselt.

2. Planare, biologisch verträgliche Makrovorrichtung auf Hydrogel-Basis nach Anspruch 1, wobei die zumindest eine Seitenwand jedes einer Vielzahl von Mikrowells weiters Gefäßendothelzellen umfasst.

3. Planare, biologisch verträgliche Makrovorrichtung auf Hydrogel-Basis nach Anspruch 1 oder 2, wobei die Beschichtung aus einem immunisolierenden Hydrogel ein Alginat-Hydrogel ist.

4. Planare, biologisch verträgliche Makrovorrichtung auf Hydrogel-Basis nach einem der Ansprüche 1 bis 3, wobei jeder der Mikrowells weiters einen Zapfen umfasst, der so angeordnet ist, das er die Bildung eines toroidförmigen Mikrogewebes lenkt.

5. Planare, biologisch verträgliche Makrovorrichtung auf Hydrogel-Basis nach einem der Ansprüche 1 bis 3, wobei das therapeutische Mikrogewebe toroidförmig ist.

6. Planare, biologisch verträgliche Makrovorrichtung auf Hydrogel-Basis nach einem der Ansprüche 1 bis 4, wobei das Mikrogewebe sekretorische, strukturelle oder metabolische Zellen umfasst.

7. Planare, biologisch verträgliche Makrovorrichtung auf Hydrogel-Basis nach einem der Ansprüche 1 bis 5, wobei das Mikrogewebe Zellen umfasst, die aus der aus Langerhans-Zellen, Hepatozyten, mononukleären Knochenmarkszellen, mesenchymalen Stammzellen, mobilisierten mononukleären Zellen des peripheren Bluts, endothelialen Vorläuferzellen, Follikelzellen, Leydig-Zellen, Ovarialzellen, neuralen Stammzellen, menschlichen embryonalen Stammzellen, pluripotenten Stammzellen, induzierten pluripotenten Stammzellen (iPSCs), Skelettmuskel-Myoblasten, Kardiomyoblasten und genetisch veränderten Zellen für Wachstumshormonmangel oder Hämophilie bestehenden Gruppe ausgewählt sind.

8. Planare, biologisch verträgliche Makrovorrichtung auf Hydrogel-Basis nach Anspruch 7, wobei das vernetzte Hydrogel aus a) GelMA ist und/oder das immunisolierende Hydrogel aus b) Alginat ist.

9. Planare, biologisch verträgliche Makrovorrichtung auf Hydrogel-Basis nach einem der Ansprüche 1 bis 8, wobei jeder der Mikrowells ein Breitenmaß im Bereich von 100 µm bis 1000 µm, vorzugsweise von 500 µm, aufweist.

10. Planare, biologisch verträgliche Makrovorrichtung auf Hydrogel-Basis nach einem der Ansprüche 4 bis 9, wobei der Zapfen ein Breitenmaß im Bereich von 50 µm bis 150 µm, vorzugsweise ein Breitenmaß von 100 µm, aufweist; und/oder
wobei der Zapfen ein Breitenmaß von 100 µm aufweist und das Breitenmaß jedes der Mikrowells 500 µm beträgt.

11. Planare, biologisch verträgliche Makrovorrichtung auf Hydrogel-Basis nach einem der Ansprüche 1 bis 10, wobei jedes der therapeutischen Mikrogewebe:
a) zu den Mikrowells zugesetzt wurde oder
b) in den Mikrowells aus einer Zellsuspension erzeugt wurde, wobei die Zellsuspension vorzugsweise etwa 0,5 bis 1,5 Millionen Zellen pro cm² der Makrovorrichtung umfasst.

12. Verfahren zur Herstellung einer planaren, biologisch verträglichen Makrovorrichtung auf Hydrogel-Basis nach einem der Ansprüche 1 bis 11, das die folgenden Schritte umfasst:
a) ein Hydrogel-Präpolymer wird auf eine Oberfläche aufgebracht;
i) eine Photomaske mit einer Anordnung von Löchern oder transparenten Elementen mit einer spezifizierten Geometrie und spezifizierten Abmessungen wird über dem aufgebrachten Gemisch platziert und
ii) das Gemisch wird UV-Licht oder einer einen Vernetzer enthaltenden Lösung ausgesetzt, um einen freiliegenden Teil des Hydrogel-Präpolymers zu vernetzen und so ein mikrostrukturiertes Netzwerk von Seitenwänden zu bilden, und
iii) die vernetzte Hydrogel-Struktur wird gespült, um nichtvernetzte Hydrogel-Rückstände zu entfernen, wodurch Mikrowells zurückbleiben; oder
b) ein Hydrogel-Präpolymer wird mithilfe von 3D-Druck auf eine Oberfläche aufgebracht und vernetzt, um ein mikrostrukturiertes Netzwerk von Seitenwänden zu bilden, die Mikrowells definieren;
c) das Zusetzen eines Gemischs aus Mikrogeweben und einem vernetzbaren immunisolierenden Hydrogel zu der Mikrostruktur aus vernetztem Hydrogel aus a) oder b) und das Vernetzen des immunisolierenden Hydrogels; oder
d) das Zusetzen einer Suspension von Zellen zu den Mikrowells und das Kultivieren der Zellen unter geeigneten Bedingungen, bis die Zellen in Abhängigkeit von der gewählten Mikrostruktur aus vernetztem Hydrogel zu einer gewünschten Form aus Mikrogewebe aggregieren, und
(i) das Zusetzen eines vernetzbaren immunisolierenden Hydrogels auf die Mikrostruktur aus vernetztem Hydrogel und
(ii) das Vernetzen des immunisolierenden Hydrogels;
wobei die planare, biologisch verträgliche Makrovorrichtung auf Hydrogel-Basis ein einzelnes Mikrogewebe pro Mikrowell umfasst;
und das weiters einen Schritt e) des Einkapselns der Makrovorrichtung und der Mikrogewebe in einer Schicht aus biologisch verträglichem Hydrogel, z. B. einer Schicht aus Alginat-Hydrogel, umfasst.

13. Verfahren nach Anspruch 12, wobei die Zellen auf der Makrovorrichtung mit etwa 0,3 bis 1,5 Millionen Zellen pro cm² der Makrovorrichtung ausgesät werden.

14. Verfahren nach einem der Ansprüche 12 oder 13, wobei in Schritt a) oder b) ein Gemisch aus einem Hydrogel-Präpolymer und Gefäßendothelzellen auf der Oberfläche verteilt wird und wobei die Gefäßendothelzellen in die Mikrowell-Seitenwände eingebettet werden.

15. Verfahren nach einem der Ansprüche 12 bis 14, wobei das Hydrogel-Präpolymer in Schritt a) oder b) GelMA ist und das vernetzte immunisolierende Hydrogel in Schritt c) oder d)(i) Alginat ist.

16. Planare, biologisch verträgliche Makrovorrichtung auf Hydrogel-Basis nach einem der Ansprüche 1 bis 11 zur Behandlung einer Erkrankung bei einem Individuum, wobei die Makrovorrichtung oder die Zusammensetzung vorzugsweise Mikrogewebe umfasst, die Insulin sekretieren, um Diabetes zu behandeln, und/oder wobei die Makrovorrichtung oder die Zusammensetzung Mikrogewebe umfasst, die Inselzellen umfassen.

17. Set, das eine planare, biologisch verträgliche Makrovorrichtung auf Hydrogel-Basis nach einem der Ansprüche 1 bis 11 umfasst.

## Revendications

1. Macrodispositif à base d'hydrogel biocompatible plan comprenant un réseau de micropuits et un microtissu thérapeutique à l'intérieur de chacun d'une pluralité desdits micropuits, dans lequel :
a) ledit macrodispositif comprend un composant comprenant un composant « verrou » d'un réseau interconnecté d'hydrogel réticulé, qui sert de parois latérales de séparation séparant des micropuits espacés de manière régulière, dans lequel chaque micropuits dans le réseau comprend au moins une paroi latérale et est configuré pour encapsuler un microtissu thérapeutique unique ; et
b) ledit macrodispositif comprend un composant « clé » comprenant des microtissus thérapeutiques dans un hydrogel immuno-isolant qui, lorsqu'ils sont réticulés, sont piégés dans les micropuits, et
dans lequel ledit composant « verrou » d'hydrogel immuno-isolant de b) se verrouille mutuellement avec le composant « clé » de réseau d'hydrogel interconnecté de a) par des caractéristiques de topographie complémentaires qui s'emboîtent dans une configuration de verrou et de clé, et
dans lequel ledit macrodispositif à base d'hydrogel biocompatible plan comprend en outre un revêtement d'un hydrogel immuno-isolant qui encapsule le macrodispositif.

2. Macrodispositif à base d'hydrogel biocompatible plan selon la revendication 1, dans lequel la au moins une paroi latérale de chaque micropuits d'une pluralité desdits micropuits comprend en outre des cellules endothéliales vasculaires.

3. Macrodispositif à base d'hydrogel biocompatible plan selon la revendication 1 ou 2, dans lequel le revêtement d'hydrogel immuno-isolant est un hydrogel d'alginate.

4. Macrodispositif à base d'hydrogel biocompatible plan selon l'une quelconque des revendications 1 à 3, dans lequel chacun desdits micropuits comprend en outre une cheville agencée pour guider la formation d'un microtissu de forme toroïdale.

5. Macrodispositif à base d'hydrogel biocompatible plan selon l'une quelconque des revendications 1 à 3, dans lequel le microtissu thérapeutique est de forme toroïdale.

6. Macrodispositif à base d'hydrogel biocompatible plan selon l'une quelconque des revendications 1 à 4, dans lequel le microtissu comprend des cellules sécrétrices, structurales ou métaboliques.

7. Macrodispositif à base d'hydrogel biocompatible plan selon l'une quelconque des revendications 1 à 5, dans lequel les microtissus comprennent des cellules choisies dans le groupe comprenant des îlots de cellules de Langerhans, des hépatocytes, des cellules mononucléées de moelle osseuse, des cellules souches mésenchymateuses, des cellules mononucléées de sang périphérique mobilisées, des cellules progénitrices endothéliales, des cellules folliculaires, des cellules de Leydig, des cellules ovariennes, des cellules souches neurales, des cellules souches embryonnaires humaines, des cellules souches pluripotentes, des cellules souches pluripotentes induites (iPSC), des myoblastes squelettiques, des cardiomyoblastes et des cellules génétiquement modifiées pour une carence en hormone de croissance ou une hémophilie.

8. Macrodispositif à base d'hydrogel biocompatible plan selon la revendication 7, dans lequel l'hydrogel réticulé en a) est du GeIMA et/ou l'hydrogel immuno-isolant en b) est de l'alginate.

9. Macrodispositif à base d'hydrogel biocompatible plan selon l'une quelconque des revendications 1 à 8, dans lequel chacun desdits micropuits présente une dimension de largeur dans la plage de 100 µm à 1000 µm, de préférence de 500 µm.

10. Macrodispositif à base d'hydrogel biocompatible plan selon l'une quelconque des revendications 4 à 9, dans lequel la cheville présente une dimension de largeur dans la plage de 50 µm à 150 µm, de préférence la cheville présente une dimension de largeur de 100 µm, et/ou
dans lequel la cheville présente une dimension de largeur de 100 µm et la dimension de largeur de chacun desdits micropuits est de 500 µm.

11. Macrodispositif à base d'hydrogel biocompatible plan selon l'une quelconque des revendications 1 à 10, dans lequel chacun desdits microtissus thérapeutiques :
a) a été ajouté auxdits micropuits, ou
b) a été généré dans lesdits micropuits à partir d'une suspension cellulaire, dans lequel de préférence la suspension cellulaire comprend environ 0,5 à 1,5 million de cellules par cm² de macrodispositif.

12. Procédé de fabrication d'un macrodispositif à base d'hydrogel biocompatible plan selon l'une quelconque des revendications 1 à 11, comprenant les étapes suivantes :
a) un prépolymère d'hydrogel est distribué sur une surface ;
i) un photomasque avec un réseau de trous ou de caractéristiques transparentes de géométrie et de dimensions spécifiées est placé sur le mélange distribué, et
ii) le mélange est exposé à une lumière UV ou à une solution contenant un agent de réticulation pour réticuler une partie exposée du prépolymère d'hydrogel, afin de former un réseau de parois latérales à micro-motif, et
iii) le motif d'hydrogel réticulé est rincé pour éliminer les résidus d'hydrogel non réticulés, en laissant derrière des micropuits ; ou
b) le prépolymère d'hydrogel est distribué sur une surface en utilisant une impression 3D et réticulé pour former un réseau à micro-motif de parois latérales définissant des micropuits ;
c) ajouter un mélange de microtissus et d'hydrogel immuno-isolant réticulable sur le micro-motif d'hydrogel réticulé de a) ou b), et réticuler l'hydrogel immuno-isolant ; ou
d) ajouter une suspension de cellules aux micropuits et cultiver les cellules dans des conditions appropriées jusqu'à ce que les cellules s'agrègent en une forme de microtissu souhaitée en fonction du micro-motif d'hydrogel réticulé choisi, et
(i) ajouter un hydrogel immuno-isolant réticulable sur le micro-motif d'hydrogel réticulé, et
(ii) réticuler l'hydrogel immuno-isolant,
dans lequel le macrodispositif à base d'hydrogel biocompatible plan comprend un microtissu unique par micropuits, et comprenant en outre une étape e) d'encapsulation du macrodispositif et des microtissus dans une couche d'hydrogel biocompatible, telle qu'une couche d'hydrogel d'alginate.

13. Procédé selon la revendication 12, dans lequel les cellules sont ensemencées sur le macrodispositif à environ 0,3 à 1,5 million de cellules par cm² de macrodispositif.

14. Procédé selon l'une quelconque des revendications 12 ou 13, dans lequel lors de l'étape a) ou b), un mélange de prépolymère d'hydrogel et de cellules endothéliales vasculaires est distribué sur ladite surface et dans lequel lesdites cellules endothéliales vasculaires sont incorporées dans les parois latérales de micropuits.

15. Procédé selon l'une quelconque des revendications 12 à 14, dans lequel le prépolymère d'hydrogel dans l'étape a) ou b) est du GeIMA et l'hydrogel immuno-isolant réticulable dans l'étape c) ou d)(i) est de l'alginate.

16. Macrodispositif à base d'hydrogel biocompatible plan selon l'une quelconque des revendications 1 à 11 pour le traitement d'une maladie chez un sujet, de préférence dans lequel le macrodispositif ou la composition comprend des microtissus qui sécrètent de l'insuline pour traiter le diabète, et/ou dans lequel le macrodispositif ou la composition comprend des microtissus qui comprennent des cellules d'îlot.

17. Kit comprenant un macrodispositif à base d'hydrogel biocompatible plan selon l'une quelconque des revendications 1 à 11.
